# EUROPEAN PATENT APPLICATION

(11) **EP 4 582 533 A1**
(43) Date of publication of application: **09.07.2025**
(21) Application number: 23860357.5
(22) Date of filing: 29.08.2023
(51) Int. Cl.: C12N 1/20, C12N 15/57, A23C 9/127

(54) **LACTIC ACID BACTERIUM COMPOSITION AND PRODUCTION METHOD OF FERMENTED MILK**

(30) Priority: 30.08.2022 JP 2022136941
(71) Applicant: MEIJI CO., LTD, Chuo-ku Tokyo 104-8306 (JP)
(72) Inventor: TSUJI Erika, Hachioji-shi, Tokyo 192-0919 (JP); GOTO Hirofumi, Hachioji-shi, Tokyo 192-0919 (JP); MAKABE Yoshimi, Hachioji-shi, Tokyo 192-0919 (JP)
(74) Representative: TBK
(86) International application number: PCT/JP2023/031264
(87) International publication number: WO 2024/048595

(57) **Abstract**

A lactic acid bacteria composition comprising: Lactobacillus delbrueckii subsp. bulgaricus; Streptococcus thermophilus carrying a prtS gene; and Streptococcus thermophilus not carrying a prtS gene.

<?xml version="1.0" encoding="UTF-8"?>
<!DOCTYPE ST26SequenceListing PUBLIC "-//WIPO//DTD Sequence Listing 1.3//EN"
"ST26SequenceListing_V1_3.dtd">
<ST26SequenceListing originalFreeTextLanguageCode="en" dtdVersion="V1_3"
fileName="IBPF23-507WO.xml" softwareName="WIPO Sequence" softwareVersion="2.2.0"
productionDate="2023-05-25">
<ApplicantFileReference>IBPF23-507WO</ApplicantFileReference>
<EarliestPriorityApplicationIdentification>
<IPOfficeCode>JP</IPOfficeCode>
<ApplicationNumberText>JP2022-136941</ApplicationNumberText>
<FilingDate>2022-08-30</FilingDate>
</EarliestPriorityApplicationIdentification>
<ApplicantName languageCode="en">Meiji Co., Ltd.</ApplicantName>
<InventionTitle languageCode="ja">LACTIC ACID BACTERIUM COMPOSITION AND
PRODUCTION METHOD OF FERMENTED MILK</InventionTitle>
<SequenceTotalQuantity>2</SequenceTotalQuantity>
<SequenceData sequenceIDNumber="1">
<INSDSeq>
<INSDSeq_length>20</INSDSeq_length>
<INSDSeq_moltype>DNA</INSDSeq_moltype>
<INSDSeq_division>PAT</INSDSeq_division>
<INSDSeq_feature-table>
<INSDFeature>

<INSDFeature_key>source</INSDFeature_key>

<INSDFeature_location>1..20</INSDFeature_location>
<INSDFeature_quals>
<INSDQualifier>

<INSDQualifier_name>mol_type</INSDQualifier_name>

<INSDQualifier_value>other DNA</INSDQualifier_value>
</INSDQualifier>
<INSDQualifier id="q2">

<INSDQualifier_name>note</INSDQualifier_name>

<INSDQualifier_value>Fprimer</INSDQualifier_value>
</INSDQualifier>
<INSDQualifier id="q3">

<INSDQualifier_name>organism</INSDQualifier_name>

<INSDQualifier_value>synthetic construct</INSDQualifier_value>
</INSDQualifier>
</INSDFeature_quals>
</INSDFeature>
</INSDSeq_feature-table>

<INSDSeq_sequence>gacttgaagaaacagcgcgt</INSDSeq_sequence>
</INSDSeq>
</SequenceData>
<SequenceData sequenceIDNumber="2">
<INSDSeq>
<INSDSeq_length>20</INSDSeq_length>
<INSDSeq_moltype>DNA</INSDSeq_moltype>
<INSDSeq_division>PAT</INSDSeq_division>

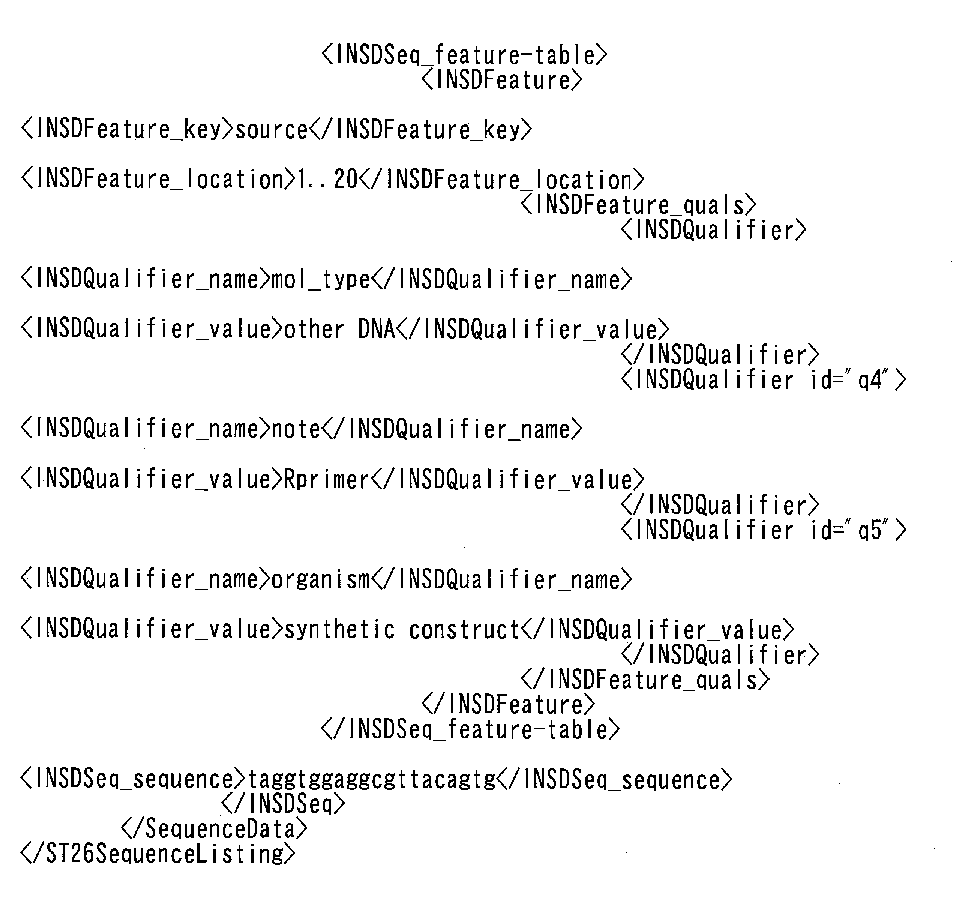

## Description

### [Technical Field]

The present invention relates to a lactic acid bacterium composition and a production method of fermented milk.

### [Background Art]

Fermented milk is defined as, for example, in Japan's "Ministerial Ordinance on Milk and Milk Products Concerning Compositional Standards, etc. (Ministerial Ordinance on Milk and Milk Products) , " "products which are obtained by fermenting milk, or milk, etc. comprising an equal or greater amount of milk solids-not-fat with lactic acid bacteria or yeast and then forming a paste or liquid, or the frozen product. " Representative examples of such fermented milk include yogurt such as set type yogurt (solid fermented milk), soft type yogurt (pasty fermented milk), and drink type yogurt (liquid fermented milk). Yogurt is defined as, for example, in the "Codex Alimentarius (FAO (Food and Agriculture Organization of the United Nations)/WHO (World Health Organization)) ," which is a food standard shared by the international community, "any food that is made through the process of lactic acid fermentation combining Lactobacillus delbrueckii subsp. bulgaricus and Streptococcus thermophilus." Conventionally, yogurt generally refers to a product obtained by adding these two types of lactic acid bacteria as a starter to raw material milk and fermenting lactose in the raw material milk; it is characterized by having sourness mainly due to lactic acid produced by the fermentation.

However, since lactic acid bacteria such as Streptococcus thermophilus can produce lactic acid by fermentation even at low temperatures, in the fermented product after the fermentation (for example, fermented milk such as yogurt), the amount of lactic acid produced becomes excessive during storage even under low temperature conditions, and the acidity increases (that is, the pH decreases), resulting in a problem that its flavor is impaired.

Therefore, several studies have been conducted to suppress acid production by such lactic acid bacteria at low temperatures. For example, Japanese Unexamined Patent Application Publication No. Hei 7-236416 (PTL 1) describes, for the purpose of obtaining fermented milk with less increase in acidity under low-temperature storage, using a strain of Lactobacillus delbrueckii subsp. bulgaricus which suppresses acid production and a strain of Streptococcus salivarius subsp. thermophilus which produces viscous substances. Japanese Unexamined Patent Application Publication No. 2000-270844 (PTL 2) describes a cold-sensitive lactic acid bacterium strain belonging to Streptococcus thermophilus that has excellent acid production ability in the temperature range of 37 to 43°C and inferior acid production ability in the temperature range of 15 to 25°C. Furthermore, Japanese Unexamined Patent Application Publication No. 2005-21050 (PTL 3) describes high acid-producing Streptococcus thermophilus KT01 strain, which shows excellent growth in a yogurt mix comprising milk as a main raw material and has a final produced lactic acid acidity of 0.9% by mass or more and less than 1% by mass when cultured at 37°C for 3 days.

### [Citation List]

### [Patent Literature]

[PTL 1] Japanese Unexamined Patent Application Publication No. Hei 7-236416
[PTL 2] Japanese Unexamined Patent Application Publication No. 2000-270844
[PTL 3] Japanese Unexamined Patent Application Publication No. 2005-21050

### [Summary of Invention]

### [Technical Problem]

Conventionally, in order to suppress acid production at low temperatures, a method of searching for and using a specific bacterial strain as described above has been mainly employed, but this requires a great deal of effort. In addition, since the flavor and physical properties of fermented milk mainly depend on the characteristics of each lactic acid bacterium, fermented milk that requires a combination limited to specific bacterial strains as in the above-mentioned related art can only provide fermented milk with limited flavors and physical properties. Moreover, when the present inventors conducted further studies, they found that, for example, when low acid-producing Streptococcus thermophilus or the like was used as in the above related art, the amount of acid produced at low temperature in the resulting fermented milk was reduced, and the decrease in pH and the increase in acidity were suppressed, but there was a problem that it took time to complete the fermentation (for example, until the pH reached 4.65). Furthermore, it has also been found that when fermented milk is stored at a low temperature, the viable cell count of Lactobacillus delbrueckii subsp. bulgaricus decreases, whereas simply increasing the viable cell count of Lactobacillus delbrueckii subsp. bulgaricus (for example, 1 × 10⁷ cfu/g or more) causes a problem that the acidity of fermented milk increases and the flavor deteriorates.

The present invention has been made in view of the above-mentioned problems of the related art, and aims to provide fermented milk in which the viable cell count of Lactobacillus delbrueckii subsp. bulgaricus is sufficiently maintained even after low-temperature storage, the decrease in pH and the increase in acidity during the low-temperature storage are also suppressed, and which has smooth physical properties, as well as a lactic acid bacteria composition and a method for producing fermented milk capable of obtaining the fermented milk in a sufficiently short fermentation time.

### [Solution to Problem]

As a result of conducting intensive research to achieve the above object, the present inventors have found that by combining Lactobacillus delbrueckii subsp. bulgaricus with Streptococcus thermophilus carrying the prtS gene and Streptococcus thermophilus not carrying the prtS gene as Streptococcus thermophilus, the viable cell count of Lactobacillus delbrueckii subsp. bulgaricus is sufficiently maintained even when the resulting fermented milk is stored at a low temperature. Furthermore, it has been found that in the fermented milk, even though the viable cell count of Lactobacillus delbrueckii subsp. bulgaricus is high, the decrease in pH and the increase in acidity during the low-temperature storage are also suppressed, and smooth physical properties are also exhibited. Furthermore, it has also been found that according to such a combination, it is possible to shorten the fermentation time, which has been difficult to achieve with the combination of two lactic acid bacteria, Lactobacillus delbrueckii subsp. bulgaricus and Streptococcus thermophilus not carrying the prtS gene, so that the above fermented milk can be obtained in a sufficiently short fermentation time. In addition, the present inventors have found that as long as the above combination is used, the three types of lactic acid bacteria are not limited to specific bacterial strains, and for example, lactic acid bacteria having various characteristics such as polysaccharide high-producing lactic acid bacteria can be employed, so that the flavor and physical properties of the fermented milk can be easily adjusted; thus, the present invention has been completed.

The aspects of the present invention obtained from such findings are as follows.
[1] A lactic acid bacteria composition comprising: Lactobacillus delbrueckii subsp. bulgaricus; Streptococcus thermophilus carrying a prtS gene; and Streptococcus thermophilus not carrying a prtS gene.
[2] The lactic acid bacteria composition according to [1] , wherein a content of the Lactobacillus delbrueckii subsp. bulgaricus and a total content of the Streptococcus thermophilus carrying the prtS gene and the Streptococcus thermophilus not carrying the prtS gene are in a mass ratio of 1:1 to 1:150.
[3] The lactic acid bacteria composition according to [1] or [2], wherein a content of the Streptococcus thermophilus carrying the prtS gene and a content of the Streptococcus thermophilus not carrying the prtS gene are in a mass ratio of 1:0.05 to 1:10.
[4] The lactic acid bacteria composition according to any one of [1] to [3], which is a composition in which, when the composition is added to a milk preparation solution comprising raw material milk and fermented at 43°C until the pH of the milk preparation solution reaches 4.65 and the post-fermentation composition is stored at 10°C for 30 days, the viable cell count of Lactobacillus delbrueckii subsp. bulgaricus in the post-fermentation composition at 30 days of storage becomes 1 x 10⁷ cfu/g or more.
[5] The lactic acid bacteria composition according to any one of [1] to [4], which is a composition in which, when the composition is added to a milk preparation solution comprising raw material milk and fermented at 43°C until the pH of the milk preparation solution reaches 4.65 and the post-fermentation composition is stored at 10°C for 30 days, the viable cell count of Lactobacillus delbrueckii subsp. bulgaricus in the post-fermentation composition at 30 days of storage becomes 10% or more of the viable cell count of Lactobacillus delbrueckii subsp. bulgaricus in the post-fermentation composition at 1 day of storage.
[6] The lactic acid bacteria composition according to any one of [1] to [5], which is a composition in which, when the composition is added to a milk preparation solution comprising raw material milk and fermented at 43°C, the time until the pH of the milk preparation solution reaches 4.65 is 5 hours or less from the start of the fermentation.
[7] The lactic acid bacteria composition according to any one of [1] to [6], which is a composition in which, when the composition is added to a milk preparation solution comprising raw material milk and fermented at 43°C, the time until the pH of the milk preparation solution reaches 4.65 is 99% or less of the time until the pH of the milk preparation solution reaches 4.65 when a lactic acid bacteria composition that comprises the Lactobacillus delbrueckii subsp. bulgaricus and the Streptococcus thermophilus not carrying the prtS gene but does not comprise the Streptococcus thermophilus carrying the prtS gene is fermented under the same conditions.
[8] The lactic acid bacteria composition according to any one of [1] to [7], which is a composition in which, when the composition is added to a milk preparation solution comprising raw material milk and fermented at 43°C until the pH of the milk preparation solution reaches 4.65 and the post-fermentation composition is stored at 10°C for 30 days, the pH of the post-fermentation composition at 30 days of storage is 4.1 or more.
[9] The lactic acid bacteria composition according to any one of [1] to [8], which is a composition in which, when the composition is added to a milk preparation solution comprising raw material milk and fermented at 43°C until the pH of the milk preparation solution reaches 4.65 and the post-fermentation composition is stored at 10°C for 30 days, the acidity of the post-fermentation composition at 30 days of storage is 1.0% or less.
[10] The lactic acid bacteria composition according to any one of [1] to [9], which is a composition in which, when the composition is added to a milk preparation solution comprising raw material milk and fermented at 43°C until the pH of the milk preparation solution reaches 4.65 and the post-fermentation composition is stored at 10°C for 30 days, the acidity (D₁%) of the post-fermentation composition at 1 day of storage and the acidity (D_{30%}) of the post-fermentation composition at 30 days of storage satisfy a condition expressed by the following formula: D₃₀ - D₁ ≤ 0.25.
[11] The lactic acid bacteria composition according to any one of [1] to [10], which is a composition in which, when the composition is added to a milk preparation solution comprising raw material milk and fermented at 43°C until the pH of the milk preparation solution reaches 4.65 and the post-fermentation composition is stored at 10°C for 30 days, a content of the Lactobacillus delbrueckii subsp. bulgaricus and a content of the Streptococcus thermophilus carrying the prtS gene and the Streptococcus thermophilus not carrying the prtS gene in the post-fermentation composition at 1 to 30 days of storage are in a viable cell count ratio of 1:2 to 1:150.
[12] The lactic acid bacteria composition according to any one of [1] to [11], which is a composition in which, when the composition is added to a milk preparation solution comprising raw material milk and fermented at 43°C until the pH of the milk preparation solution reaches 4.65 and the post-fermentation composition is stored at 10°C for 30 days, a content of the Streptococcus thermophilus carrying the prtS gene and a content of the Streptococcus thermophilus not carrying the prtS gene in the post-fermentation composition at 1 to 30 days of storage are in a viable cell count ratio of 1:0.005 to 1:30.
[13] The lactic acid bacteria composition according to any one of [1] to [12], wherein the Lactobacillus delbrueckii subsp. bulgaricus and/or the Streptococcus thermophilus not carrying the prtS gene are/is a high polysaccharide-producing lactic acid bacteria/bacterium.
[14] The lactic acid bacteria composition according to any one of [1] to [13], which is a lactic acid bacteria starter.
[15] The lactic acid bacteria composition according to any one of [1] to [13], which is a fermented milk.
[16] A lactic acid bacteria combination comprising: Lactobacillus delbrueckii subsp. bulgaricus; Streptococcus thermophilus carrying a prtS gene; and Streptococcus thermophilus not carrying a prtS gene.
[17] The lactic acid bacteria combination according to [16], which is a lactic acid bacteria starter.
[18] A method for producing fermented milk, comprising a fermentation step of adding the lactic acid bacteria composition according to any one of [1] to [15] or the lactic acid bacteria combination according to [16] or [17] to a milk preparation solution comprising raw material milk and fermenting the mixture to obtain a fermented milk.

### [Advantageous Effects of Invention]

The present invention makes it possible to provide fermented milk in which the viable cell count of Lactobacillus delbrueckii subsp. bulgaricus is sufficiently maintained even after low-temperature (for example, 10°C) storage, the decrease in pH and the increase in acidity during the low-temperature storage are also suppressed, and which has smooth physical properties, as well as a lactic acid bacteria composition and a method for producing fermented milk capable of obtaining the fermented milk in a sufficiently short fermentation time.

### [Description of Embodiments]

The present invention will now be described in detail with reference to preferred embodiments thereof.

### (Lactic Acid Bacteria)

In the present invention, the "lactic acid bacteria (or lactic acid bacterium)" is a general term for microorganisms capable of assimilating glucose and producing 50% or more of lactic acid in sugar-based yield, and physiological properties thereof include Gram-positive cocci or bacilli, non-motile, often non-spore-forming (although some lactic acid bacteria such as Bacillus coagulans are capable of spore formation), and catalase-negative. The lactic acid bacteria according to the present invention are lactic acid bacteria belonging to Lactobacillus delbrueckii subsp. bulgaricus and Streptococcus thermophilus.

### (Lactobacillus delbrueckii subsp. bulgaricus)

Lactobacillus delbrueckii subsp. bulgaricus is a subspecies of lactic acid bacteria of the genus Lactobacillus belonging to lactic acid bacteria of the family Lactobacillaceae. In the present specification, Lactobacillus delbrueckii subsp. bulgaricus is sometimes referred to as "L. bulgaricus."

Examples of the L. bulgaricus according to the present invention include bacterial strains such as Lactobacillus delbrueckii subsp. bulgaricus OLL1171 identified by accession number NITE BP-01569 (hereinafter sometimes referred to as "OLL1171 strain"), Lactobacillus delbrueckii subsp. bulgaricus OLL1073R-1 identified by accession number FERM BP-10741 (hereinafter sometimes referred to as "OLL1073R-1 strain"), Lactobacillus delbrueckii subsp. bulgaricus OLL205013 identified by accession number NITE BP-02411 (hereinafter sometimes referred to as "OLL205013 strain"), Lactobacillus delbrueckii subsp. bulgaricus OLL1247 identified by accession number NITE BP-01814 (hereinafter sometimes referred to as "OLL1247 strain"), Lactobacillus delbrueckii subsp. bulgaricus OLL1251 identified by accession number NITE BP-02703 (hereinafter sometimes referred to as "OLL1251 strain"), and Lactobacillus delbrueckii subsp. bulgaricus 1589 identified by accession number NITE BP-03716 (hereinafter sometimes referred to as "1589 strain"), but is not limited thereto.

The OLL1171 strain has (1) identification label: Lactobacillus delbrueckii subsp. bulgaricus OLL1171, (2) accession number: NITE BP-01569, and (3) date of accession: March 13, 2013; the OLL1073R-1 strain has (1) identification label: Lactobacillus delbrueckii subsp. bulgaricus OLL1073R-1, (2) accession number: FERM BP-10741, and (3) date of accession: February 22, 1999; the OLL205013 strain has (1) identification label: Lactobacillus delbrueckii subsp. bulgaricus OLL205013, (2) accession number: NITE BP-02411, and (3) date of accession: February 3, 2017; the OLL1247 strain has (1) identification label: Lactobacillus delbrueckii subsp. bulgaricus OLL1247, (2) accession number: NITE BP-01814, and (3) date of accession: March 6, 2014; the OLL1251 strain has (1) identification label: Lactobacillus delbrueckii subsp. bulgaricus OLL1251, (2) accession number: NITE BP-02703, and (3) date of accession: April 25, 2018; and the 1589 strain has (1) identification label: Lactobacillus delbrueckii subsp. bulgaricus 1589, (2) accession number: NITE BP-03716, and (3) date of accession: August 9, 2022; strains labeled "NITE" have been deposited at (4) depositary institution: NITE Patent Microorganisms Depositary, National Institute of Technology and Evaluation (postal code 292-0818, Room 122, 2-5-8 Kazusa Kamatari, Kisarazu City, Chiba Prefecture); strains labeled "FERM" have been deposited at (4) depositary institution: International Patent Organism Depositary, National Institute of Technology and Evaluation (postal code 292-0818, Room 120, 2-5-8 Kazusa Kamatari, Kisarazu City, Chiba Prefecture; however, at the time of deposit for the OLL1073R-1 strain: International Patent Organism Depositary, National Institute of Advanced Industrial Science and Technology (postal code 305-8566, 1-1-1 Higashi, Tsukuba City, Ibaraki Prefecture, Central 6)). The L. bulgaricus specified by these accession numbers may be a subcultured strain of the same strain, or an artificial mutant strain, a natural mutant strain, a genetically modified strain, or a derivative strain of the same strain or a subcultured strain thereof, as long as the effects of the present invention are not impaired.

The L. bulgaricus according to the present invention may also be, for example, a L. bulgaricus isolated from commercially available fermented milk using a known agar medium for the genus Lactobacillus (for example, MRS agar medium or BCP-supplemented plate count agar medium). Such a L. bulgaricus includes, for example, the L. bulgaricus 2038 strain isolated from "Meiji Bulgaria Yogurt (registered trademark) , manufactured by Meiji Co., Ltd." with a spindle-like colony shape on BCP-supplemented plate count agar medium, which is stored at Meiji Innovation Center of Meiji Co., Ltd. (postal code 192-0919, 1-29-1 Nanakuni, Hachioji City, Tokyo, Japan).

### [High Polysaccharide-Producing L. bulgaricus]

As the L. bulgaricus according to the present invention, L. bulgaricus having various characteristics can be employed, and for example, from the viewpoint of improving the viscosity of fermented milk, it is preferably a high polysaccharide-producing lactic acid bacterium. In the present specification, the "high polysaccharide-producing lactic acid bacterium" refers to a lactic acid bacterium capable of producing a large amount of exopolysaccharide (EPS).

Whether or not the L. bulgaricus is a high polysaccharide-producing lactic acid bacterium can be confirmed by, for example, the method described in the Examples below. Specifically, for example, by measuring the amount of exopolysaccharide (EPS) in a culture medium in which the L. bulgaricus after activation as necessary has been cultured (for example, after two activation cultures of a frozen bacterial strain in a 10 w/v% skim milk medium, the resulting cultured solution is adjusted to 1 platinum loop/5 mL and is then inoculated into a fresh 10 w/v% skim milk medium for stationary culture at 37°C for 18 hours, so that the final concentration becomes 1 w/w%), if the amount is large, it can be determined that the L. bulgaricus is a high polysaccharide-producing lactic acid bacterium. The amount of exopolysaccharide (EPS) can be appropriately measured by a conventionally known method, for example, a method of measuring the amount of polysaccharide obtained by dissolving the components in 4 mL of the culture medium with trichloroacetic acid, then precipitating and recovering the polysaccharide with ethanol, and dialyzing with ultrapure water as necessary, by the phenol-sulfuric acid method. For example, it can be determined that the L. bulgaricus is a high polysaccharide-producing lactic acid bacterium when the amount of polysaccharide (mg/kg) in the culture medium measured by the above method is 70 mg/kg or more, 105 mg/kg or more, 116 mg/kg or more, or 120 mg/kg or more. Further, for example, if the amount of polysaccharide (mg/kg) measured by the above method exceeds the amount of exopolysaccharide (EPS) in the culture medium obtained by culturing the L. bulgaricus 2038 strain, more specifically, if it exceeds 1.0 times, or is 1.1 times or more, of the amount of exopolysaccharide (EPS) in the culture medium obtained by culturing the L. bulgaricus 2038 strain, it may be determined that the L. bulgaricus is a particularly high polysaccharide-producing lactic acid bacterium.

Specific examples of the L. bulgaricus that is such a high polysaccharide-producing lactic acid bacterium include, but are not limited to, the OLL1251 strain, the OLL1073R-1 strain, and the OLL1247 strain.

As the L. bulgaricus according to the present invention, one type may be used alone, or two or more types may be used in combination.

### (Streptococcus thermophilus)

Streptococcus thermophilus, also called thermophilus bacterium, is a chain-forming coccus capable of producing lactic acid from lactose. In the present specification, lactic acid bacteria belonging to Streptococcus thermophilus are sometimes referred to as "S. thermophilus."

In the present invention, regarding S. thermophilus, it is necessary to combine S. thermophilus carrying a prtS gene and S. thermophilus not carrying a prtS gene. In the present invention, the "prtS gene" refers to a gene encoding a cell wall-associated serine protease that degrades casein. Whether S. thermophilus has the prtS gene in the present invention can be confirmed, for example, by the method described in Examples below. Specifically, for example, a highly conserved sequence is selected among the prtS genes of S. thermophilus which can be obtained from public databases (such as Genbank), and a portion of the prtS gene is amplified by PCR method using a primer set prepared based on the sequence; if a desired PCR product is obtained, it can be determined that it has the prtS gene. Examples of the primer set include, but are not limited to, the primer set consisting of Fprimer (SEQ ID NO: 1) and Rprimer (SEQ ID NO: 2) described in Examples below.

### [S. thermophilus (prtS+) Carrying prtS Gene]

Examples of the S. thermophilus carrying the prtS gene according to the present invention include, but are not limited to, Streptococcus thermophilus OLS4801 identified by accession number NITE BP-03504 (hereinafter sometimes referred to as "OLS4801 strain"), Streptococcus thermophilus OLS4802 identified by accession number NITE BP-03505 (hereinafter sometimes referred to as "OLS4802 strain") , Streptococcus thermophilus OLS4803 identified by accession number NITE BP-03506 (hereinafter sometimes referred to as "OLS4803 strain"), Streptococcus thermophilus OLS4823 identified by accession number NITE BP-03507 (hereinafter sometimes referred to as "OLS4823 strain"), and Streptococcus thermophilus OLS4824 identified by accession number NITE BP-03508 (hereinafter sometimes referred to as "OLS4824 strain").

The OLS4801 strain has (1) identification label: Streptococcus thermophilus OLS4801, (2) accession number: NITE BP-03504, and (3) date of accession: August 4, 2021; the OLS4802 strain has (1) identification label: Streptococcus thermophilus OLS4802, (2) accession number: NITE BP-03505, and (3) date of accession: August 4, 2021; the OLS4803 strain has (1) identification label: Streptococcus thermophilus OLS4803, (2) accession number: NITE BP-03506, and (3) date of accession: August 4, 2021; the OLS4823 strain has (1) identification label: Streptococcus thermophilus OLS4823, (2) accession number: NITE BP-03507, and (3) date of accession: August 4, 2021; and the OLS4824 strain has (1) identification label: Streptococcus thermophilus OLS4824, (2) accession number: NITE BP-03508, and (3) date of accession: August 4, 2021; they have been deposited at (4) depositary institution: NITE Patent Microorganisms Depositary, National Institute of Technology and Evaluation (postal code 292-0818, Room 122, 2-5-8 Kazusa Kamatari, Kisarazu City, Chiba Prefecture). The S. thermophilus specified by these accession numbers may be a subcultured strain of the same strain, or may be an artificial mutant, a natural mutant, a genetically modified strain, or a derivative strain of the same strain or a subculture strain thereof, within a range that does not inhibit the effects of the present invention.

The S. thermophilus carrying the prtS gene according to the present invention may also be, for example, S. thermophilus isolated as a large yellow colony from commercially available fermented milk using an agar medium for the genus Lactococcus (for example, fast-slow differential agar (FSDA, hereinafter sometimes referred to as "FS agar medium")). The FS agar medium is preferably a plate medium prepared by preparing solution A: 3.8 g of disodium glycerophosphate pentahydrate (final concentration: 1.9 w/w%), 0.008 g of bromocresol purple (final concentration: 0.004 w/w%), 3 g of agar (final concentration: 1.5 w/w%), and 120 g of ultrapure water, and solution B: 20 g of skim milk powder (final concentration: 10 w/w%) and 80 g of ultrapure water, pasteurizing them (for example, solution A by autoclave pasteurization at 121°C for 7 minutes, and solution B by autoclave pasteurization at 110°C for 10 minutes), cooling them (for example, to about 55°C), and mixing solution A and solution B so that the concentration of each component becomes the final concentration. The culture in the FS agar medium is preferably performed, for example, by surface-streaking 100 µL of a sample diluted 10⁶ times with physiological saline and culturing at 37°C for 24 hours.

As the S. thermophilus carrying the prtS gene according to the present invention, one type may be used alone, or two or more types may be used in combination.

### [S. thermophilus (prtS-) Not Carrying prtS Gene]

Examples of the S. thermophilus not carrying the prtS gene according to the present invention include, but are not limited to, Streptococcus thermophilus OLS3078 identified by accession number NITE BP-01697 (hereinafter sometimes referred to as "OLS3078 strain"), Streptococcus thermophilus OLS3615 identified by accession number NITE BP-01696 (hereinafter sometimes referred to as "OLS3615 strain"), and Streptococcus thermophilus OLS3290 identified by accession number FERM BP-19638 (hereinafter sometimes referred to as "OLS3290 strain").

The OLS3078 strain has (1) identification label: Streptococcus thermophilus OLS3078, (2) accession number: NITE BP-01697, and (3) date of accession: August 23, 2013; the OLS3615 strain has (1) identification label: Streptococcus thermophilus OLS3615, (2) accession number: NITE BP-01696, and (3) date of accession: August 23, 2013; and the OLS3290 strain has (1) identification label: Streptococcus thermophilus OLS3290, (2) accession number: FERM BP-19638, and (3) date of accession: January 19, 2004; strains labeled "NITE" have been deposited at (4) depositary institution: NITE Patent Microorganisms Depositary, National Institute of Technology and Evaluation (postal code 292-0818, Room 122, 2-5-8 Kazusa Kamatari, Kisarazu City, Chiba Prefecture); strains labeled "FERM" have been deposited at (4) depositary institution: International Patent Organism Depositary, National Institute of Technology and Evaluation (postal code 292-0818, Room 120, 2-5-8 Kazusa Kamatari, Kisarazu City, Chiba Prefecture). The S. thermophilus specified by these accession numbers may be a subcultured strain of the same strain, or may be an artificial mutant strain, a natural mutant strain, a genetically modified strain, or a derivative strain of the same strain or a subcultured strain thereof, as long as the effects of the present invention are not impaired.

The S. thermophilus not carrying the prtS gene according to the present invention may also be, for example, S. thermophilus isolated as a small, flat, translucent colony from commercially available fermented milk using an agar medium for the genus Lactococcus (for example, FS agar medium). Such an S. thermophilus not carrying the prtS gene includes, for example, the S. thermophilus 1131 strain, which is isolated from "Meiji Bulgaria Yogurt (registered trademark) LB81, manufactured by Meiji Co., Ltd." using M17 agar medium, BCP-supplemented plate count agar, or FS agar medium, and is kept at the Meiji Innovation Center of Meiji Co., Ltd. (postal code 192-0919, 1-29-1 Nanakuni, Hachioji City, Tokyo, Japan). When the S. thermophilus 1131 strain is isolated on a BCP-supplemented plate count agar medium, it is isolated as a spherical colony.

As the S. thermophilus not carrying the prtS gene according to the present invention, one type may be used alone, or two or more types may be used in combination.

### [High Polysaccharide-Producing S. thermophilus]

As the S. thermophilus according to the present invention, particularly the S. thermophilus not carrying the prtS gene, S. thermophilus having various characteristics can be employed, and for example, from the viewpoint of improving the viscosity of fermented milk, it is preferably the high polysaccharide-producing lactic acid bacterium.

Whether or not S. thermophilus is a high polysaccharide-producing lactic acid bacterium can be confirmed by, for example, the method described in the Examples below. Specifically, for example, by measuring the amount of exopolysaccharide (EPS) in the culture medium obtained by culturing the S. thermophilus after activation as necessary (for example, after two activation cultures of a frozen bacterial strain in a 10 w/v% skim milk - 0.1 w/v% casein-decomposed peptide medium, the resulting cultured solution is adjusted to 1 platinum loop/5 mL and is then inoculated into a fresh 10 w/v% skim milk - 0.1 w/v% casein-decomposed peptide medium for stationary culture at 43°C for 4 hours, so that the final concentration becomes 1 w/w%), if the amount is large, it can be determined to be a high polysaccharide-producing lactic acid bacterium. The amount of exopolysaccharide (EPS) can be appropriately measured by a conventionally known method, for example, a method of measuring the amount of polysaccharide obtained by dissolving components in 10 g of the culture medium with trichloroacetic acid, then precipitating and collecting the polysaccharide with ethanol, and dialyzing with ultrapure water if necessary, by the phenol-sulfuric acid method. For example, when the amount of polysaccharide (mg/kg) in the culture medium measured by the above method is 3 mg/kg or more, 12 mg/kg or more, 13 mg/kg or more, or 14 mg/kg or more, it can be determined that S. thermophilus is a high polysaccharide-producing lactic acid bacterium. Further, for example, if the amount of polysaccharide (mg/kg) measured by the above method exceeds the amount of exopolysaccharide (EPS) in the culture medium obtained by culturing the S. thermophilus 1131 strain, more specifically, if it exceeds 1.0 times, or is 1.1 times or more, of the amount of exopolysaccharide (EPS) in the culture medium obtained by culturing the S. thermophilus 1131 strain, it may be determined to be a particularly high polysaccharide-producing lactic acid bacterium.

Specific examples of such S. thermophilus that is such a high polysaccharide-producing lactic acid bacterium include, but are not limited to, the OLS3290 strain and the OLS3078 strain.

### (Lactic Acid Bacteria Composition and Lactic Acid Bacteria Combination)

The lactic acid bacteria composition (or lactic acid bacterium composition) of the present invention comprises L. bulgaricus, S. thermophilus carrying the prtS gene, and S. thermophilus not carrying the prtS gene. When the lactic acid bacteria composition of the present invention is the lactic acid bacteria starter described below, the lactic acid bacteria composition is a lactic acid bacteria combination (or lactic acid bacterium combination) comprising L. bulgaricus, S. thermophilus carrying the prtS gene, and S. thermophilus not carrying the prtS gene (that is, a lactic acid bacteria starter kit). In this case, the combination ratio and total amount of L. bulgaricus, S. thermophilus carrying the prtS gene, and S. thermophilus not carrying the prtS gene preferably correspond to the mass ratio or viable cell count ratio and total content or total viable cell count in the lactic acid bacteria composition of the present invention, respectively.

In the lactic acid bacteria composition of the present invention, these three lactic acid bacteria (L. bulgaricus, S. thermophilus carrying the prtS gene, and S. thermophilus not carrying the prtS gene) acquire a new symbiotic relationship so that the viable cell count of the L. bulgaricus is sufficiently maintained even after low-temperature storage, the decrease in pH and the increase in acidity during the low-temperature storage are also suppressed, and fermented milk having smooth physical properties can be obtained in a sufficiently short fermentation time, the present inventors presume.

In the lactic acid bacteria composition of the present invention, the content of L. bulgaricus (content of viable cells of L. bulgaricus, the same shall apply hereinafter) is preferably 0.5 to 30% by mass, more preferably 0.8 to 15% by mass, and further preferably 1 to 10% by mass, based on the overall mass (total mass) of viable cells of the three lactic acid bacteria.

In the lactic acid bacteria composition of the present invention, the content of S. thermophilus carrying the prtS gene (content of viable cells of S. thermophilus carrying the prtS gene, the same shall apply hereinafter) is preferably 15 to 80% by mass, more preferably 18 to 80% by mass, and further preferably 20 to 75% by mass, based on the overall mass (total mass) of viable cells of the three lactic acid bacteria.

In the lactic acid bacteria composition of the present invention, the content of S. thermophilus not carrying the prtS gene (content of viable cells of S. thermophilus not carrying the prtS gene, the same shall apply hereinafter) is preferably 10 to 75% by mass, more preferably 10 to 72% by mass, and further preferably 10 to 70% by mass, based on the overall mass (total mass) of viable cells of the three lactic acid bacteria.

In the lactic acid bacteria composition of the present invention, the content of L. bulgaricus, the content of S. thermophilus carrying the prtS gene, and the content of S. thermophilus not carrying the prtS gene (content of L. bulgaricus: content of S. thermophilus carrying the prtS gene: content of S. thermophilus not carrying the prtS gene) may be combined as appropriate; examples include a combination of 0.5 to 30% by mass: 15 to 80% by mass: 10 to 75% by mass, a combination of 0.8 to 15% by mass: 18 to 80% by mass: 10 to 72% by mass, a combination of 1 to 10% by mass: 20 to 75% by mass: 10 to 70% by mass, a combination of 0.8 to 15% by mass or 1 to 10% by mass: 15 to 48% by mass: 48 to 75% by mass, and a combination of 0.8 to 15% by mass or 1 to 10% by mass: 48 to 75% by mass: 15 to 48% by mass, based on the overall mass (total mass) of the viable cells of the three lactic acid bacteria.

More preferably, in the lactic acid bacteria composition of the present invention, the ratio of the content of the L. bulgaricus to the total of the content of the S. thermophilus carrying the prtS gene and the content of the S. thermophilus not carrying the prtS gene is preferably 1:1 to 1:150, more preferably 1:2 to 1:120, and even more preferably 1:5 to 1:100, in terms of mass ratio (mass of viable cells of L. bulgaricus: total mass of viable cells of S. thermophilus).

Furthermore, in the lactic acid bacteria composition of the present invention, the ratio of the content of S. thermophilus carrying the prtS gene to the content of S. thermophilus not carrying the prtS gene is preferably 1:0.05 to 1:10, more preferably 1:0.075 to 1:8, and even more preferably 1:0.1 to 1:6, in terms of mass ratio (mass of viable cells of S. thermophilus carrying the prtS gene: mass of viable cells of S. thermophilus not carrying the prtS gene).

The lactic acid bacteria composition of the present invention may further comprise other components than the three types of lactic acid bacteria according to the present invention; the other components are not particularly limited, and include, for example, solvents such as water; cultures such as supernatant of culture medium and medium components after activation culture of lactic acid bacteria; post-fermentation composition after fermentation of the lactic acid bacteria composition; concentrates, dilutions, dried products, frozen products, and the like of the cultures and the post-fermentation compositions, and may be used singly or in combination of two or more.

In the case of a lactic acid bacteria combination, the three types of lactic acid bacteria according to the present invention may be a composition comprising one or two of them, and in this case, the composition may further comprise other components than lactic acid bacteria. Examples of the other components are the same as described above.

### [Fermentation Time]

The lactic acid bacteria composition of the present invention (including the lactic acid bacteria starter and the fermented milk described below, preferably the lactic acid bacteria starter) preferably satisfies the following condition (b):
(b) when added to a milk preparation solution comprising raw material milk and fermented at 43°C, the time until the pH of the milk preparation solution reaches 4.65 is 99% or less of the time until the pH of the milk preparation solution reaches 4.65 when a lactic acid bacteria composition that comprises L. bulgaricus and S. thermophilus not carrying the prtS gene but does not comprise S. thermophilus carrying the prtS gene is fermented under the same conditions (hereinafter referred to as fermentation time with two lactic acid bacteria).

More specifically, it is preferable that it satisfies the condition that when added to a milk preparation solution comprising raw material milk and fermented at 43°C, the time until the pH of the milk preparation solution reaches 4.65 becomes 75% or less of the fermentation time with the two lactic acid bacteria if the fermentation time with the two lactic acid bacteria is 6 hours or more, 90% or less if the fermentation time with the two lactic acid bacteria is 4 hours or more and less than 6 hours, and 99% or less if the fermentation time with the two lactic acid bacteria is 3 hours or more and less than 4 hours. It is also more preferable to satisfy the condition
(a) when added to a milk preparation solution comprising raw material milk and fermented at 43°C, the time until the pH of the milk preparation solution reaches 4.65 becomes 5 hours or less from the start of the fermentation.

### <Activation Culture>

In conditions (a) to (b) and conditions (c) to (i) below, it is preferable to first activate and culture the lactic acid bacteria composition (or each lactic acid bacterium constituting the lactic acid bacteria composition) after low-temperature (for example, 10°C or lower) storage or frozen storage. The activation culture is preferably performed in a skim milk medium at 37°C under aerobic conditions for 16 to 18 hours (preferably 16 hours). At this time, the amount of lactic acid bacteria used for the activation culture is preferably an amount that makes the amount of each lactic acid bacterium relative to the skim milk medium 1 platinum loop/5 mL, more specifically, an amount that makes the viable cell count 1 × 10² to 2 × 10⁸ cfu/g, more preferably an amount that makes the viable cell count 1 × 10⁴ to 1 × 10⁶ cfu/g.

In the present invention, the viable cell count is measured, for example, by appropriately diluting a properly-diluted solution comprising the lactic acid bacteria composition (or each lactic acid bacterium constituting the lactic acid bacteria composition) (for example, culture medium of activation culture (lactic acid bacteria and skim milk medium)), spreading it on a suitable agar medium (for example, in the case of L. bulgaricus, BCP-supplemented plate count agar medium, which is an official medium specified by the Ministerial Ordinance on Milk and Milk Products; in the case of S. thermophilus, FS agar medium), culturing, and counting the number of colonies that appear. At this time, the L. bulgaricus forms spindle-shaped colonies in the BCP-supplemented plate count agar medium, and S. thermophilus carrying the prtS gene forms large yellow colonies in the FS agar medium, whereas S. thermophilus not carrying the prtS gene forms small, flat, translucent colonies in the FS agar medium, so that they can be distinguished.

In the present invention, the term "skim milk medium" refers to a medium comprising 10 w/w% of skim milk powder and 0.1 w/w% of yeast extract in ultrapure water. The skim milk powder according to the present invention refers to skim milk obtained by removing fat from animal milk (preferably cow's milk) and dried into powder form; for example, in Japan's "Ministerial Ordinance on Milk and Milk Products Concerning Compositional Standards, etc. (hereinafter sometimes referred to as "Ministerial Ordinance on Milk and Milk Products")," skim milk powder is specified to have a milk solids-not-fat content of 95.0% or more and a moisture content of 5.0% or less. The composition of such skim milk powder includes, for example, a composition including the composition described in "Standard Tables of Food Composition in Japan - 2015 - (Seventh Revised Edition)," that is, per 100 g: energy 300 to 400 (preferably 359) kcal, moisture 3 to 5 (preferably 3.8) g, protein 30 to 40 (preferably 34.0) g, fat 0.2 to 2 (preferably 1.0) g, carbohydrate (including lactose) 45 to 60 (preferably 53.3) g, calcium 900 to 2000 (preferably 1100) mg, vitamin A 3 to 10 (preferably 6) µg, vitamin B₂ 0.5 to 2 (preferably 1.60) mg, cholesterol 10 to 30 (preferably 25) mg, and salt equivalent 0.5 to 2 (preferably 1.4) g. The pH of such a skim milk medium is usually 6 to 7.

The skim milk medium may further comprise other components (for example, peptides) in addition to the skim milk powder and yeast extract, but even in that case, the content of the other components is more preferably 0.01% by mass or less. When the activation culture is performed two or more times, the skim milk medium used for the second and subsequent activation cultures may comprise a part or all of the culture medium after the previous culture. The skim milk medium is preferably sterilized or pasteurized.

The method for the activation culture is not particularly limited, but includes stationary culture under conditions conventionally known as aerobic culture conditions for lactic acid bacteria. In the present invention, the activation culture may be repeated multiple times (preferably twice) by inoculating the culture medium after the above culture again into the skim milk medium so that the culture medium after the culture becomes, for example, 1 w/w%.

### <Fermentation>

In conditions (a) to (b) and conditions (c) to (i) below, the lactic acid bacteria composition of the present invention after the activation culture, or the lactic acid bacteria composition of the present invention obtained by mixing each lactic acid bacterium after the activation culture, is added to a milk preparation solution and fermented at 43°C until the pH of the milk preparation solution reaches 4.65, to obtain a post-fermentation composition.

The amount of lactic acid bacteria used for fermentation according to conditions (a) to (b) and conditions (c) to (i) below is preferably an amount that makes the total viable cell count of the three types of lactic acid bacteria according to the present invention 1 × 10⁵ to 5 × 10⁹ cfu/g, and more preferably an amount that makes the total viable cell count 1 × 10⁶ to 2 × 10⁹ cfu/g, relative to the milk preparation solution.

In the present invention, the "milk preparation solution" refers to a liquid comprising raw material milk. The raw material milk preferably comprises lactose, and examples thereof include raw milk (for example, milk of cows, water buffalos, sheep, goats, etc.), pasteurized milk, whole milk, skim milk, whey, and processed products thereof (for example, whole milk powder, full-fat concentrated milk, skim milk powder, skim concentrated milk, condensed milk, whey powder, buttermilk, butter, cream, cheese, whey protein concentrate (WPC), whey protein isolate (WPI), α-lactalbumin (α-La), and β-lactoglobulin (β-Lg)), which may be any one of these or a mixture of two or more.

The milk preparation solution according to the present invention may be composed only of the above raw material milk, or may be an aqueous solution, diluted solution, or concentrated solution of the above raw material milk, or may further comprise other components as necessary in addition to the above raw material milk; however, the milk preparation solution according to conditions (a) to (b) and conditions (c) to (i) below is preferably composed of cow's milk, skim milk powder, and ion-exchanged water. Furthermore, the milk preparation solution according to each of the above conditions preferably has a fat content of 0 to 4.0 w/w% (preferably 3.05 w/w%) and a milk solids-not-fat content of 8.0 to 12.0 w/w% (preferably 9.7 w/w%). The pH of such a milk preparation solution is usually 6 to 7.

The milk preparation solution can be prepared by mixing the above components while heating and/or homogenizing as necessary. The milk preparation solution is preferably sterilized or pasteurized.

The fermentation method is not particularly limited, and includes stationary culture under conditions conventionally known as fermentation conditions using lactic acid bacteria.

In the fermentation, when fermentation occurs in which lactic acid is produced from lactose comprised in the milk preparation solution by the metabolism of lactic acid bacteria, the pH of the milk preparation solution decreases due to the lactic acid. In the fermentation according to the conditions (a) to (b) and (c) to (i) below, the completion is based on the pH of the milk preparation solution (that is, the pH of the milk preparation solution comprising the lactic acid bacteria composition) reaching 4.65.

In condition (a), the time until completion of the fermentation (that is, until the pH of the milk preparation solution reaches 4.65) needs to be 5 hours or less, more preferably 4.5 hours or less, and even more preferably 4 hours or less, from the start of fermentation. If the time until the completion of the fermentation exceeds the above upper limit, it is not preferable in terms of production efficiency because it takes time for production when used in the production of fermented milk. The lower limit of the time until completion of the fermentation is not particularly limited, but is preferably 1 hour or more, and more preferably 2 hours or more. Note that even in conventional lactic acid bacteria compositions, condition (a) and/or condition (b) below may be satisfied, but in such cases, for example, the viable cell count of L. bulgaricus is reduced due to low-temperature storage (does not satisfy condition (c) and/or (d)), or the pH decreases or the acidity increases due to low-temperature storage (does not satisfy at least one of conditions (e) to (g)).

In condition (b), the time until completion of the fermentation (that is, until the pH of the milk preparation solution reaches 4.65) needs to be 99% or less of the time until completion of fermentation of a lactic acid bacteria composition obtained by removing S. thermophilus carrying the prtS gene from the lactic acid bacteria composition of the present invention (that is, a lactic acid bacteria composition that comprises L. bulgaricus and S. thermophilus not carrying the prtS gene but does not comprise S. thermophilus carrying the prtS gene, the same shall apply hereinafter) (fermentation time with two lactic acid bacteria); more specifically, the time until the completion of the fermentation is more preferably 75% or less of the fermentation time with the two lactic acid bacteria if the fermentation time with the two lactic acid bacteria is 6 hours or more, 90% or less if the fermentation time with the two lactic acid bacteria is 4 hours or more and less than 6 hours, and 99% or less if the fermentation time with the two lactic acid bacteria is 3 hours or more and less than 4 hours. In particular, when the time until the completion of fermentation of the lactic acid bacteria composition obtained by removing S. thermophilus having the prtS gene from the lactic acid bacteria composition of the present invention is long (for example, 7 hours or longer), in the lactic acid bacteria composition of the present invention, it is possible to shorten the time to 70% or less, and further to 65% or less.

### [Viable Cell Count of L. bulgaricus]

The lactic acid bacteria composition of the present invention (including the lactic acid bacteria starter and the fermented milk below, preferably the lactic acid bacteria starter) preferably satisfies at least one of the following conditions (c) and (d), more preferably both conditions:
(c) when added to a milk preparation solution comprising raw material milk and fermented at 43°C until the pH of the milk preparation solution becomes 4.65 and the post-fermentation composition is stored at 10°C for 30 days, the viable cell count of the L. bulgaricus in the post-fermentation composition after 30 days of storage is 1 × 10⁷ cfu/g or more;
(d) when added to a milk preparation solution comprising raw material milk and fermented at 43°C until the pH of the milk preparation solution becomes 4.65 and the post-fermentation composition is stored at 10°C for 30 days, the viable cell count of the L. bulgaricus in the post-fermentation composition after 30 days of storage is 10% or more of the viable cell count of the L. bulgaricus in the post-fermentation composition after 1 day of storage.

### <Storage>

In conditions (c) and (d) and conditions (e) to (i) below, the post-fermentation composition after the fermentation is complete (that is, after the pH reaches 4.65) is cooled and stored. The storage conditions are not particularly limited, and include methods known as methods for storing fermented milk, such as a method of sealing (preferably hermetically sealing) the post-fermentation composition in a container and leaving to stand.

Conditions (c) and (d) and conditions (e) to (i) below preferably do not include a step of rapidly decreasing the pH. An example of the step of rapidly decreasing the pH is the addition of intentionally added acid (e.g., lactic acid).

The storage temperature according to conditions (c) and (d) and conditions (e) to (i) below needs to be low, and more specifically, 10°C. Note that an error of ±1°C is permissible. The storage period according to these conditions is 30 days. In the lactic acid bacteria composition of the present invention, the viable cell count of the L. bulgaricus is sufficiently maintained even if it is stored under low temperature conditions for a long period.

Condition (c) requires that the viable cell count of the L. bulgaricus in the post-fermentation composition after 30 days of storage at 10°C is 1 × 10⁷ cfu/g or more, more preferably 2 × 10⁷ cfu/g or more, and even more preferably 2.5 × 10⁷ cfu/g or more.

Condition (d) requires that the viable cell count of the L. bulgaricus in the post-fermentation composition after 30 days of storage at 10°C is 10% or more, more preferably 15% or more, even more preferably 20% or more, still more preferably 23% or more, and particularly preferably 30% or more, of the viable cell count of the L. bulgaricus in the post-fermentation composition after 1 day of storage.

### [pH and Acidity]

The lactic acid bacteria composition of the present invention (including the lactic acid bacteria starter and fermented milk described below, preferably lactic acid bacteria starter) preferably satisfies at least one of the following conditions (e) to (g), more preferably two or more, even more preferably all conditions:
(e) when added to a milk preparation solution comprising raw material milk and fermented at 43°C until the pH of the milk preparation solution becomes 4.65 and the post-fermentation composition is stored at 10°C for 30 days, the pH of the post-fermentation composition after 30 days of storage is 4.1 or more;
(f) when added to a milk preparation solution comprising raw material milk and fermented at 43°C until the pH of the milk preparation solution becomes 4.65 and the post-fermentation composition is stored at 10°C for 30 days, the acidity of the post-fermentation composition after 30 days of storage is 1.0% or less;
(g) when added to a milk preparation solution comprising raw material milk and fermented at 43°C until the pH of the milk preparation solution becomes 4.65 and the post-fermentation composition is stored at 10°C for 30 days, the acidity (D₁%) of the post-fermentation composition at 1 day of storage and the acidity (D₃₀%) of the post-fermentation composition at 30 days of storage satisfy the condition expressed by the following formula: D₃₀ - D₁ ≤ 0.25.

Lactic acid bacteria (particularly S. thermophilus) are usually capable of producing lactic acid even at a low temperature, so that if the post-fermentation composition after the completion of fermentation is stored for a long period of time as described above, even under low temperature conditions, the pH of the post-fermentation composition further decreases as the production amount of lactic acid increases, and the acidity further increases. In contrast, in the lactic acid bacteria composition of the present invention, the decrease in pH and the increase in acidity during the low-temperature storage are sufficiently suppressed.

Condition (e) requires that the pH of the post-fermentation composition (that is, the pH of the milk preparation solution comprising the lactic acid bacteria composition) after 30 days of storage at 10°C is 4.1 or more, more preferably 4.12 or more, even more preferably 4.14 or more.

Condition (f) requires that the acidity of the post-fermentation composition (that is, lactic acid acidity (w/v%) of the milk preparation solution comprising the lactic acid bacteria composition, the same applies hereinafter) after 30 days of storage at 10°C is 1.0% or less, more preferably 0.98 or less, even more preferably 0.96 or less.

Condition (g) requires that the acidity (D₁%) of the post-fermentation composition after 1 day of storage at 10°C and the acidity (D₃₀%) of the post-fermentation composition after 30 days of storage satisfy a condition expressed by the following formula: D₃₀ - D₁ ≤ 0.25; the value of D₃₀ - D₁ is more preferably 0.23 or less, even more preferably 0.20 or less, still more preferably 0.19 or less.

Even conventional lactic acid bacteria compositions, for example, comprising lactic acid bacteria which are said to be cold-sensitive, may not decrease the pH of post-fermentation composition even after storing for a long time at low temperature. However, in this case, it takes a long time to complete fermentation (does not satisfy conditions (a) and/or (b)), or the viable cell count of L. bulgaricus is reduced due to low-temperature storage (does not satisfy conditions (c) and/or (d)).

### [Viable Cell Count of S. thermophilus]

The lactic acid bacteria composition of the present invention (including the lactic acid bacteria starter and fermented milk below, preferably the lactic acid bacteria starter) also preferably satisfies at least one of the following conditions (h) and (i), more preferably both conditions:
(h) when added to a milk preparation solution comprising raw material milk and fermented at 43°C until the pH of the milk preparation solution becomes 4.65 and the post-fermentation composition is stored at 10°C for 30 days, the content of the L. bulgaricus and the content of S. thermophilus carrying the prtS gene and S. thermophilus not carrying the prtS gene in the post-fermentation composition at 1 to 30 days of storage are in a viable cell count ratio of 1:2 to 1:150;
(i) when added to a milk preparation solution comprising raw material milk and fermented at 43°C until the pH of the milk preparation solution becomes 4.65 and the post-fermentation composition is stored at 10°C for 30 days, the content of S. thermophilus carrying the prtS gene and the content of S. thermophilus not carrying the prtS gene in the post-fermentation composition for 1 to 30 days of storage are in a viable cell count ratio of 1:0.005 to 1:30.

In condition (h), the ratio of the viable cell count of the L. bulgaricus to the total viable cell count of S. thermophilus carrying the prtS gene and S. thermophilus not carrying the prtS gene in the post-fermentation composition at 1 to 30 days of storage at 10°C needs to be 1:2 to 1:150, more preferably 1:3 to 1:140, even more preferably 1:5 to 1:140, still more preferably 1:10 to 1:130, and also preferably 1:3 to 1:60.

In condition (i), the ratio of the viable cell count of S. thermophilus carrying the prtS gene to the viable cell count of S. thermophilus not carrying the prtS gene in the post-fermentation composition at 1 to 30 days of storage at 10°C needs to be 1:0.005 to 1:30, more preferably 1:0.010 to 1:20, even more preferably 1:0.015 to 1:10, and also preferably 1:0.03 to 1:15.

### [Lactic Acid Bacteria Starter]

The lactic acid bacteria composition and lactic acid bacteria combination of the present invention satisfy at least one of the above conditions (a) to (i) (preferably two conditions of (c) or (d) and (e) or (f) or (g); more preferably four conditions of (a) or (b), (c) or (d), (e) or (f) or (g), and (h) or (i); even more preferably at least five conditions of (c) to (g); still more preferably all conditions of (a) to (i)), so that they can be suitably used, for example, as a lactic acid bacteria starter (or lactic acid bacterium starter) for the method for producing fermented milk of the present invention described below. Thereby, the viable cell count of L. bulgaricus is sufficiently maintained even after low-temperature storage, the decrease in pH and the increase in acidity during the low-temperature storage are also suppressed, and fermented milk also having smooth physical properties can be obtained in a short time.

The lactic acid bacteria starter according to the present invention may be a composition further comprising or a combination including other lactic acid bacteria and yeast, as long as the effects of the present invention are not impaired. Examples of the other lactic acid bacteria and yeast include lactic acid bacteria and yeast which are conventionally known to be comprised in fermented milk. Examples of the other lactic acid bacteria include lactic acid bacteria of the genus Lactobacillus other than L. bulgaricus (for example, Lactobacillus delbrueckii other than L. bulgaricus, such as Lactobacillus delbrueckii subsp. delbrueckii, Lactobacillus delbrueckii subsp. lactis, Lactobacillus delbrueckii subsp. indicus, Lactobacillus delbrueckii subsp. sunkii, and Lactobacillus delbrueckii subsp. jakobsenii; Lactobacillus helveticus; Lactobacillus acidophilus; Lactobacillus johnsonii; Lactobacillus gasseri; Lactobacillus paragasseri; Lactobacillus amylovorus; and Lactobacillus crispatus, etc.), lactic acid bacteria of the genus Lacticaseibacillus, lactic acid bacteria of the genus Lactiplantibacillus, lactic acid bacteria of the genus Liquorilactobacillus, lactic acid bacteria of the genus Latilactobacillus, lactic acid bacteria of the genus Ligilactobacillus, lactic acid bacteria of the genus Limosilactobacillus, lactic acid bacteria of the genus Lentilactobacillus, lactic acid bacteria of the genus Levilactobacillus, lactic acid bacteria of the genus Pediococcus, lactic acid bacteria of the genus Leuconostoc, and lactic acid bacteria of the genus Lactococcus, which may be used singly or in combination of two or more.

The lactic acid bacteria starter according to the present invention may be in a liquid state, or in a solid state such as frozen or dry powder form, and may further comprise other components. Examples of further components which can be comprised in the lactic acid bacteria starter include fermentation-promoting substances (such as formic acid and nucleic acids), protective agents (such as saccharides), and medium components (such as milk and whey), which may be used singly or in combination of two or more.

When the lactic acid bacteria composition of the present invention is a lactic acid bacteria starter, the total content of the three types of lactic acid bacteria according to the present invention is preferably 0.01 to 100% by mass, more preferably 0.1 to 90% by mass. The total viable cell count of the three lactic acid bacteria is preferably 1 × 10⁶ cfu/g or more, more preferably 1 × 10⁶ to 2 × 10¹¹ cfu/g, even more preferably 1 × 10⁷ to 1 × 10¹¹ cfu/g.

The lactic acid bacteria starter according to the present invention may be, for example, as described above, a combination including L. bulgaricus or a composition comprising the same, S. thermophilus carrying the prtS gene or a composition comprising the same, and S. thermophilus not carrying the prtS gene or a composition comprising the same, such as a lactic acid bacteria starter kit. In this case, for example, additives for producing fermented milk (the above fermentation-promoting substances, protective agents, etc.), containers, instructions for use of lactic acid bacteria starter, etc. may be combined and included in the kit.

### [Fermented Milk]

The lactic acid bacteria composition of the present invention also includes fermented milk. The fermented milk according to the present invention can be obtained as the post-fermentation composition by, for example, fermentation according to conditions (a) to (i) above (that is, addition to the milk preparation solution and fermentation at 43°C until the pH of the milk preparation solution reaches 4.65), and preferably satisfies at least one of the conditions (a) to (i). In the fermented milk according to the present invention, the viable cell count of L. bulgaricus is sufficiently maintained even after low-temperature storage, the decrease in pH and the increase in acidity during low-temperature storage are also suppressed, and it also has smooth physical properties. The fermented milk according to the present invention may also be used as the above lactic acid bacteria starter for the method for producing fermented milk of the present invention described below.

The fermented milk according to the present invention includes, for example, fermented milk that satisfies the standards for "fermented milk" in the Ministerial Ordinance on Milk and Milk Products (more specifically, one having a milk solids-not-fat content of 8.0% or more and a lactic acid bacteria count or yeast count (preferably a lactic acid bacteria count (more preferably the total count of the three types of lactic acid bacteria according to the present invention), hereinafter the same) of 10 million/mL or more). The fermented milk according to the present invention includes those satisfying the standards of "milk products and fermented milk drinks" in accordance with the Ministerial Ordinance on Milk and Milk Products (more specifically, content of non-fat milk solids is not less than 3.0%, the number of viable lactic acid bacteria or yeasts is not less than 10 million/mL); it also includes those meeting the standards for "fermented milk drinks" (more specifically, content of non-fat milk solids is less than 3.0%, the number of viable lactic acid bacteria or yeasts is not less than 1 million/mL). The milk solids-not-fat refers to the remaining components (mainly protein, lactose, minerals, etc.) obtained by subtracting fat from total milk solids, and the lactic acid bacteria count and yeast count are the number of viable cells measured by the test method specified in the Ministerial Ordinance on Milk and Milk Products.

The fermented milk according to the present invention preferably has a viable cell count of L. bulgaricus of 1 × 10⁷ cfu/g or more, more preferably 2 × 10⁷ cfu/g or more, even more preferably 2.5 × 10⁷ cfu/g or more.

In the fermented milk according to the present invention, the ratio of the content of L. bulgaricus to the total content of S. thermophilus carrying the prtS gene and S. thermophilus not carrying the prtS gene is preferably 1:1 to 1:150, more preferably 1:2 to 1:150, still more preferably 1:5 to 1:140, and particularly preferably 1:10 to 1:130, in terms of viable cell count ratio (viable cell count of L. bulgaricus : total viable cell count of S. thermophilus).

Furthermore, in the fermented milk according to the present invention, the ratio of the content of S. thermophilus carrying the prtS gene to the content of S. thermophilus not carrying the prtS gene is preferably 1:0.005 to 1:30, more preferably 1:0.010 to 1:20, and even more preferably 1:0.015 to 1:10, still more preferably 1:0.05 to 1:10, in terms of viable cell count ratio (viable cell count of S. thermophilus carrying prtS gene : viable cell count of S. thermophilus not carrying prtS gene).

Furthermore, the fermented milk according to the present invention preferably has a pH of 4.1 or more, more preferably 4.12 or more, and further preferably 4.14 or more. The fermented milk according to the present invention preferably has a lactic acid acidity of 1.0% or less, more preferably 0.98 or less, and even more preferably 0.96 or less.

The fermented milk according to the present invention has smooth physical properties, and such smooth physical properties are preferably maintained even after long-term storage (for example, 20 days or 30 days) at low temperatures (for example, 10°C).

In the present invention, the "smooth physical properties" means that the texture is uniform and no roughness is felt in sensory evaluation; for example, if the roughness felt on the tongue is less than that of fermented milk obtained under the same conditions by using two lactic acid bacteria, L. bulgaricus 2038 strain and OLS4802 strain carrying the prtS gene, it can be determined to be smooth. In the present invention, the "smooth physical properties" can also be confirmed, for example, by a sufficiently low syneresis rate. Specifically, for example, when fermented milk (preferably immediately after fermentation) is stored at 10°C for 20 days, when the syneresis rate of the fermented milk at 5 days of storage (S₅%) and the syneresis rate of the fermented milk at 20 days of storage (S₂₀%) satisfy the condition expressed by the following formula: S₂₀ - S₅ ≤ 6.5 (preferably S₂₀ - S₅ ≤ 6.0), it can be determined that it is smooth. In the present invention, the "syneresis rate" is a value (%) calculated by weighing 40 g of fermented milk, centrifuging it at 1,610 × g and 25°C for 10 minutes, removing the supernatant, measuring the weight of the precipitate, and using the following formula: weight of precipitate (g)/40 g × 100.

The fermented milk according to the present invention may be the post-fermentation composition itself, or may be a product obtained by concentrating, diluting, drying, or freezing the composition.

The fermented milk according to the present invention comprises components derived from the raw material milk (preferably milk preparation solution). Further, within a range not impairing the effects of the present invention, it may further comprise the other components mentioned for the above lactic acid bacteria composition and the lactic acid bacteria starter, or other lactic acid bacteria and yeasts. Furthermore, it may comprise various components that can be comprised in food and drink. Examples of further components that can be comprised in fermented milk include, but are not limited to, saccharides, sugar alcohols, minerals, vitamins, proteins, peptides, amino acids, organic acids, pH adjusters, starches and modified starches, dietary fibers, fruits, vegetables and processed products thereof, animal and plant crude drug extracts, natural polymers (collagen, hyaluronic acid, chondroitin, etc.), oils and/or fats, thickeners, emulsifiers, solvents, surfactants, gelling agents, stabilizers, buffering agents, suspending agents, thickening agents, excipients, disintegrants, binders, fluidizing agents, preservatives, colorants, flavors, corrigents, and sweeteners, and these may be used singly or in combination of two or more.

Such fermented milk is preferably yogurt, cheese, fermented cream, fermented butter, and the like, and particularly preferably yogurt. Specific examples of the yogurt include set type yogurt (solid fermented milk) such as plain yogurt, soft type yogurt (paste-like fermented milk), and drink type yogurt (liquid fermented milk), and may be frozen yogurt using these as materials. The fermented milk according to the present invention can also be used as an ingredient for fermented foods such as cheese, fermented cream, fermented butter, and kefir.

### (Method for Producing Fermented Milk)

The method for producing fermented milk (or production method of fermented milk) of the present invention (sometimes simply referred to as "production method of the present invention") includes a fermentation step of adding the lactic acid bacteria composition or the lactic acid bacteria combination of the present invention to a milk preparation solution comprising raw material milk and fermenting the mixture to obtain a fermented milk. According to the production method of the present invention, by fermenting raw material milk using the lactic acid bacteria composition or lactic acid bacteria combination of the present invention, it is possible to suppress the decrease in pH and the increase in acidity during low-temperature storage, and the viable cell count of the comprised L. bulgaricus is sufficiently maintained even after the low-temperature storage, thereby enabling the production of fermented milk with smooth physical properties. Such smooth physical properties tend to be maintained even after long-term storage (for example, 20 days or 30 days) at a low temperature (for example, 10°C). Furthermore, the production method of the present invention makes it possible to sufficiently shorten the time required for the completion of fermentation.

### [Milk Preparation Solution]

Examples of the milk preparation solution used in the production method of the present invention include the milk preparation solution according to conditions (a) to (i) for the lactic acid bacteria composition, but the milk preparation solution according to the production method of the present invention is not limited thereto, and those conventionally known as milk preparation solutions for the production of fermented milk may also be appropriately employed.

For example, the milk preparation solution may further comprise other components; such other components include, for example, water; foods, food components, or food additives such as soymilk, saccharides including sugar, sweeteners, flavorings, fruit juices, pulps, vitamins, minerals, oils and fats, ceramides, collagen, milk phospholipids, and polyphenols; stabilizers such as pectin, soy polysaccharides, CMC (carboxymethyl cellulose), agar, gelatin, carrageenan, and gums; thickeners, gelling agents, and the like, and may be used singly or in a mixture of two or more thereof.

### [Fermentation]

In the production method of the present invention, examples of the fermentation step of adding the lactic acid bacteria composition or the lactic acid bacteria combination of the present invention to the milk preparation solution and fermenting the mixture include the fermentation according to conditions (a) to (i) for the lactic acid bacteria composition, but the fermentation according to the production method of the present invention is not limited thereto; fermentation methods conventionally known for fermented milk production may also be appropriately employed.

For example, the amount of the lactic acid bacteria composition or lactic acid bacteria combination of the present invention to be added to the milk preparation solution can be appropriately set in accordance with the amount of the lactic acid bacteria starter added that is employed in conventionally known fermented milk production methods; for example, an amount that makes the total viable cell count of the three lactic acid bacteria according to the present invention in the range of 1 × 10⁵ to 5 × 10⁹ cfu/g, preferably 1 × 10⁶ to 5 × 10⁹ cfu/g, more preferably 1 × 10⁷ to 5 × 10⁹ cfu/g, even more preferably 1 × 10⁸ to 2 × 10⁹ cfu/g, based on the milk preparation solution. In addition to the lactic acid bacteria composition or the lactic acid bacteria combination of the present invention, the other lactic acid bacteria or yeast may be further added to the milk preparation solution.

The fermentation conditions according to the production method of the present invention can be appropriately selected depending on the growth conditions of the added lactic acid bacteria, the amount of the milk preparation solution, etc., and are not particularly limited; for example, the fermentation is preferably performed under aerobic or anaerobic conditions at 35 to 45°C, more preferably at 38 to 43°C, by leaving to stand or stirring (preferably leaving to stand) for usually 2 to 16 hours, more preferably 2.5 to 8 hours, even more preferably 3 to 6 hours, until the pH of the milk preparation solution to which the lactic acid bacteria composition of the present invention has been added becomes 4.7 or less, more preferably 4.45 to 4.65. According to the present invention, it is possible to sufficiently shorten the time required for fermentation. When anaerobic conditions are employed, fermentation under nitrogen aeration conditions can be employed.

Fermented milk can be obtained by the above fermentation. The fermented product after the fermentation step can be used as fermented milk as it is or after concentrating, diluting, drying, freezing, or the like as needed. The fermented milk thus obtained is preferably fermented milk as the lactic acid bacteria composition of the present invention; however, the fermented milk obtained by the production method of the present invention does not necessarily have to be fermented milk as the lactic acid bacteria composition of the present invention due to the adoption of conditions known as a fermented milk production method, for example, addition of the other components, fermentation conditions, or processing. Also, in the production method of the present invention, fermented milk may be obtained by crushing or heat-treating the lactic acid bacteria in the fermented product, or by concentrating, diluting, drying, freezing, or the like as necessary. Therefore, the method for producing fermented milk of the present invention may further include these steps (such as concentration step, dilution step, drying step, freezing step, crushing step, and heat treatment step).

### [Examples]

Hereinafter, the present invention will be described more specifically based on Examples and Comparative Examples, but the present invention is not limited to the following Examples.

### <Lactic Acid Bacteria>

The lactic acid bacterial strains used in the following Examples and Comparative Examples are as follows.

### (L. bulgaricus 2038 strain)

Lactobacillus delbrueckii subsp. bulgaricus, stored at the Meiji Innovation Center of Meiji Co., Ltd. (postal code 192-0919, 1-29-1 Nanakuni, Hachioji City, Tokyo, Japan). Lactobacillus delbrueckii subsp. bulgaricus that can be isolated from "Meiji Bulgaria Yogurt (registered trademark), manufactured by Meiji Co., Ltd." using an agar medium for the genus Lactobacillus.

### (L. bulgaricus 1589 strain)

Lactobacillus delbrueckii subsp. bulgaricus identified by accession number NITE BP-03716. Not a high polysaccharide-producing lactic acid bacterium.

### (L. bulgaricus OLL205013 strain)

Lactobacillus delbrueckii subsp. bulgaricus identified by accession number NITE BP-02411. Not a high polysaccharide-producing lactic acid bacterium.

### (L. bulgaricus OLL1073R-1 strain)

Lactobacillus delbrueckii subsp. bulgaricus identified by accession number FERM BP-10741. A high polysaccharide-producing lactic acid bacterium having a higher polysaccharide content measured by the following method than L. bulgaricus 2038 strain.

### (L. bulgaricus OLL1251 strain)

Lactobacillus delbrueckii subsp. bulgaricus identified by accession number NITE BP-02703. A high polysaccharide-producing lactic acid bacterium having a higher polysaccharide content measured by the following method than L. bulgaricus 2038 strain.

### (L. bulgaricus OLL1171 strain)

Lactobacillus delbrueckii subsp. bulgaricus identified by accession number NITE BP-01569. Not a high polysaccharide-producing lactic acid bacterium.

### (L. bulgaricus OLL1247 strain)

Lactobacillus delbrueckii subsp. bulgaricus identified by accession number NITE BP-01569. A high polysaccharide-producing lactic acid bacterium having a higher polysaccharide content measured by the following method than L. bulgaricus 2038 strain.

### (S. thermophilus OLS4801 strain)

Streptococcus thermophilus identified by accession number NITE BP-03504. Has the prtS gene. Not a high polysaccharide-producing lactic acid bacterium.

### (S. thermophilus OLS4802 strain)

Streptococcus thermophilus identified by accession number NITE BP-03505. Has the prtS gene. Not a high polysaccharide-producing lactic acid bacterium.

### (S. thermophilus OLS4803 strain)

Streptococcus thermophilus identified by accession number NITE BP-03506. Has the prtS gene. Not a high polysaccharide-producing lactic acid bacterium.

### (S. thermophilus 1131 strain)

Lactobacillus delbrueckii subsp. bulgaricus stored at the Meiji Innovation Center of Meiji Co., Ltd. (postal code 192-0919, 1-29-1 Nanakuni, Hachioji City, Tokyo, Japan). Streptococcus thermophilus that can be isolated from "Meiji Bulgaria Yogurt (registered trademark) LB81, manufactured by Meiji Co., Ltd." using an agar medium for the genus Lactococcus. Does not have the prtS gene.

### (S. thermophilus OLS3290 strain)

Streptococcus thermophilus identified by accession number FERM BP-19638. Does not have the prtS gene. A high polysaccharide-producing lactic acid bacterium having a higher polysaccharide content measured by the following method than S. thermophilus 1131 strain.

### (S. thermophilus OLS3078 strain)

Streptococcus thermophilus identified by accession number: NITE BP-01697. Does not have the prtS gene. A high polysaccharide-producing lactic acid bacterium having a higher polysaccharide content measured by the following method than S. thermophilus 1131 strain.

### (S. thermophilus OLS3615 strain)

Streptococcus thermophilus identified by accession number: NITE BP-01696. Does not have the prtS gene. Not a high polysaccharide-producing lactic acid bacterium.

### (1) prtS Gene

In this specification, the presence or absence of the prtS gene in each Streptococcus thermophilus was confirmed by the following method. That is, first, the prtS gene sequences of five S. thermophilus strains whose genomic sequences are known were obtained from the NCBI database, and a primer set (Fprimer: SEQ ID NO: 1, Rprimer: SEQ ID NO: 2) was prepared from highly conserved sequences. Genomic DNA was extracted from the M17 culture solution of the five strains using InstaGene Matrix (manufactured by BioRad). 0.5 µL of the extracted genomic DNA (template), 1 µL each of the prepared primers (5 µM), 0.1 µL of Phusion high fidelity DNA polymerase, 2 µL of 5×HF buffer, 0.8 µL of 2.5 mM dNTPs, and 4.6 µL of ultrapure water were mixed (10 µL in total), and PCR was performed under the following conditions: 98°C for 30 seconds; 30 cycles of 98°C for 5 seconds, 63°C for 20 seconds, and 72°C for 20 seconds; 72°C for 5 minutes; and left standing at 4°C. The obtained PCR product was subjected to agarose gel electrophoresis, and bacterial strains in which a band was confirmed at the position of 684 bp were determined to have the prtS gene, and bacterial strains in which the band was not confirmed were determined not to have the prtS gene. Hereinafter, S. thermophilus carrying the prtS gene is sometimes referred to as "prtS+," and S. thermophilus not carrying the prtS gene is sometimes referred to as "prtS-."

### (2) High Polysaccharide-Producing Lactic Acid Bacteria

In this example, whether each Lactobacillus delbrueckii subsp. bulgaricus (L. bulgaricus) and Streptococcus thermophilus (S. thermophilus) is a high polysaccharide-producing lactic acid bacterium was confirmed by the following methods, respectively.

### (L. bulgaricus)

The L. bulgaricus was first activated by inoculating a frozen bacterial strain into a 10 w/v% skim milk medium (prepared by adding 10 w/v% skim milk powder to ultrapure water and sterilizing by autoclave sterilization at 121°C for 7 minutes, pH: 6 to 7, the same shall apply hereinafter) at 1 platinum loop/5 mL, culturing by leaving to stand at 37°C under aerobic conditions for 16 hours, and then again inoculating the culture medium into a 10 w/v% skim milk medium at 1 w/w% and culturing it under the same conditions. Next, 4 mL of the skim milk medium in a 15 mL conical tube was inoculated with the activated L. bulgaricus culture solution at 1 w/w%, and the mixture was cultured by leaving to stand at 37°C for 18 hours.

400 µL (final concentration: 10%) of 100% trichloroacetic acid (manufactured by FUJIFILM Wako Pure Chemical Corporation) was added to 4 mL of the culture solution after culturing, and the mixture was stirred until its viscosity disappeared; then, the supernatant was collected by centrifugation (12,000 g, 4°C, 20 minutes), and the whole amount was transferred to a new 15 mL conical tube. Ultrapure water was added to the collected supernatant, and the volume was brought up to 4 mL with a scale on the conical tube; then, 8 mL (2 times the amount) of cold ethanol was gradually added and thoroughly mixed, and after that, the mixture was left to stand overnight at 4°C to precipitate the polysaccharides. The polysaccharides were collected as a precipitate by centrifugation and completely dissolved by adding 4 mL of ultrapure water. 180 µL/well of the obtained polysaccharide aqueous solution was applied to a dialysis container (96-well dispo DIALYZER: 250-300 µ; MWCO 5,000; HARVARD 74-0902), and dialysis was performed against 5 L of ultrapure water for 2 days (ultrapure water was replaced every day). After dialysis, the volume of the polysaccharide aqueous solution was measured, and the concentration of polysaccharide was measured by the phenol-sulfuric acid method, thereby calculating the amount of polysaccharide in the culture medium (mg/kg). In this example, a L. bulgaricus having a higher polysaccharide amount than L. bulgaricus 2038 strain and a polysaccharide amount of 105 mg/kg or more was treated as a high polysaccharide-producing lactic acid bacterium.

### (S. thermophilus)

S. thermophilus was first activated by inoculating a frozen bacterial strain into a 10 w/v% skim milk - 0.1 w/v% casein decomposed peptide medium (prepared by adding 10 w/v% skim milk powder and 0.1 w/v% casein decomposed peptide (CE90GMM, manufactured by Nippon Shinyaku Co., Ltd.) to ultrapure water and pasteurizing the mixture by reaching a temperature of 95°C, pH: 6 to 7, the same shall apply hereinafter) at 1 platinum loop/5 mL, culturing by leaving to stand at 37°C under aerobic conditions for 16 hours, and then inoculating the culture solution into a 10 w/v% skim milk - 0.1 w/v% casein decomposed peptide medium again at 1 w/w%, and culturing under the same conditions. Next, 15 mL of the peptide-added medium in a 50 mL conical tube was inoculated with the activated S. thermophilus culture medium at 1 w/w%, and the mixture was cultured by leaving to stand at 43°C for 4 hours.

10 g of the culture product after culturing was weighed, 1 mL (final concentration: 10%) of 100% trichloroacetic acid (manufactured by FUJIFILM Wako Pure Chemical Corporation) was added, and the mixture was stirred until the viscosity disappeared; then, the supernatant was collected by centrifugation (12,000 g, 4°C, 20 minutes), and the entire amount was transferred to a new 50 mL conical tube. Twice the volume of cold ethanol was gradually added to the collected supernatant and thoroughly mixed, and the mixture was left standing overnight at 4°C to precipitate the polysaccharide. The polysaccharide was collected as a precipitate by centrifugation and completely dissolved by adding 10 mL of ultrapure water. The obtained polysaccharide aqueous solution was placed in a dialysis container (MWCO: 6,000-8,000; SPECTRUM), and dialyzed against 100 times the volume of ultrapure water for 2 days (ultrapure water was replaced daily). After the dialysis, the volume of the polysaccharide aqueous solution was measured, the concentration of the polysaccharide was measured by the phenol-sulfuric acid method, and the amount of the polysaccharide in the culture (mg/kg) was calculated. In this Example, S. thermophilus having a higher amount of polysaccharide than that of the S. thermophilus 1131 strain and 12 mg/kg or more was treated as a high polysaccharide-producing lactic acid bacterium.

### <Example 1>

### (1) Yogurt Preparation

First, L. bulgaricus 2038 strain, S. thermophilus OLS4802 strain carrying the prtS gene, and S. thermophilus 1131 strain not carrying the prtS gene were each activated by inoculating a frozen bacterial strain into a skim milk medium (prepared by adding 10 w/w% skim milk powder and 0.1 w/w% yeast extract to ultrapure water and sterilizing by autoclave sterilization at 121°C for 7 minutes, pH: 6 to 7, the same applies hereinafter) at 1 platinum loop/5 mL, stationary-culturing at 37°C under aerobic conditions for 16 hours, and then inoculating the culture solution into the skim milk medium again at 1 w/w% and culturing under the same conditions.

Next, a milk preparation solution (prepared by mixing cow milk, skim milk powder, and ion-exchanged water so as to have a fat content of 3.05 w/w% and a solids-not-fat content of 9.7 w/w%, and sterilizing the mixture by autoclave sterilization at 75°C for 15 minutes, pH: 6 to 7, the same applies hereinafter) was inoculated with the activated lactic acid bacteria so that the total mass becomes 3 w/w% (L. bulgaricus 2038 strain: 0.3 w/w% (viable cell count: 3.0 × 10⁵ to 9.0 × 10⁷ cfu/g), S. thermophilus OLS4802 strain: 2.1 w/w% (viable cell count: 2.1 × 10⁶ to 6.3 × 10⁸ cfu/g), S. thermophilus 1131 strain: 0.6 w/w% (viable cell count: 6.0 × 10⁵ to 1.8 × 10⁸ cfu/g)), and fermented by leaving to stand at 43°C, and the time from the inoculation (fermentation time) until the pH of the milk preparation solution decreased and reached 4.65 was measured. The fermentation time was 3 hours and 24 minutes. Furthermore, when the pH reached 4.65, the fermentation was terminated (leaving to stand at 43°C was terminated, the same applies hereinafter) to obtain yogurt.

### (2) Yogurt Analysis

The yogurt prepared in (1) above was sealed so as not to allow air to enter, left at 10°C, and stored. The pH (10°C pH) and acidity (lactic acid acidity: % (w/v%, hereinafter the same)) after 1 day and 30 days from the start of storage were measured by the following methods, and also the viable cell count (cfu/g) of L. bulgaricus and the viable cell counts (cfu/g) of each S. thermophilus were measured. The results are shown in Table 1 below.

### (pH)

The pH was measured by the glass electrode method using a pH meter HM-30R (manufactured by Mettler Toledo) .

### (Acidity)

9.0 g was collected from each yogurt after storage, 500 µL of phenolphthalein indicator was added, and the mixture was measured by neutralization titration using 0.1N NaOH with the end point being where a slight pink color does not disappear within 30 seconds.

### (Viable Cell Count of L. bulgaricus)

Using BCP-added plate count agar, an official medium for the measurement of viable cell count of lactic acid bacteria (manufactured by Eiken Chemical Co., Ltd.), each yogurt after storage was cultured at 37°C under aerobic conditions for 72 hours, and the number of formed colonies was counted. Note that in the agar medium, colonies having spindle shapes were determined to be colonies of L. bulgaricus.

### (Viable Cell Count of S. thermophilus)

First, solution A was prepared to have the following composition: 3.8 g of disodium glycerophosphate pentahydrate, 0.008 g of bromocresol purple, 3 g of agar, and 120 g of ultrapure water. Also, solution B was prepared to have the following composition: 20 g of skim milk powder and 80 g of ultrapure water. Next, solution A was sterilized by autoclave pasteurization at 121°C for 7 minutes, and solution B was sterilized by autoclave pasteurization at 110°C for 10 minutes; after cooling each to 55°C, solutions A and B were mixed so that each component had the following concentration: disodium glycerophosphate pentahydrate 1.9 w/w%, bromocresol purple 0.004 w/w%, agar 1.5 w/w%, and skim milk powder 10 w/w%, and the mixture was dispensed into plates to prepare FS agar medium.

100 µL of a sample obtained by diluting each yogurt after storage 10⁶ times with physiological saline was spread on the surface of the FS agar medium, cultured at 37°C for 24 hours, and then the number of formed colonies was counted. Note that large yellow colonies formed on the agar medium were determined to be colonies of S. thermophilus carrying the prtS gene, and small, flat, translucent colonies were determined to be colonies of S. thermophilus not carrying the prtS gene.

**[Table 1]**

| Example | L. bulgaricus | prtS+ | prtS- | Storage Period | pH | Acidity (%) | Viable Cell Count (cfu/g) | | |
|---|---|---|---|---|---|---|---|---|---|
| | | | | | | | L. bulgaricus | prtS+ | prtS- |
| 1 | 2038 Strain | OLS4802 Strain | 1131 Strain | 1 Day | 4.49 | 0.77 | 1.1×10⁸ | 2.0×10⁸ | 2.0×10⁸ |
| | | | | 30 Days | 4.14 | 0.96 | 2.6×10⁷ | 5.6×10⁸ | 2.0×10⁸ |

### <Examples 11 to 16>

Each yogurt was prepared in the same manner as in Example 1 except that the bacterial strains used in combination as L. bulgaricus, S. thermophilus carrying the prtS gene (prtS+), and S. thermophilus not carrying the prtS gene (prtS-) were as shown in Table 2 below, and yogurt analysis (measurement of pH and acidity after 1 day and 30 days from the start of storage, measurement of the viable cell count of L. bulgaricus and the viable cell counts of each S. thermophilus) was performed. The results are shown in Table 2 below.

**[Table 2]**

| Example | L. bulgaricus | prtS+ | prtS- | Storage Period | pH | Acidity (%) | Viable Cell Count (cfu/g) | | |
|---|---|---|---|---|---|---|---|---|---|
| | | | | | | | L. bulgaricus | prtS+ | prtS- |
| 11 | 1589 Strain | OLS4801 Strain | OLS3290 Strain | 1 Day | 4.50 | 0.81 | 2.7×10⁷ | 1.4×10⁹ | 9.5×10⁷ |
| | | | | 30 Days | 4.15 | 0.98 | 3.6×10⁷ | 4.3×10⁸ | 3.5×10⁷ |
| 12 | | OLS4802 Strain | | 1 Day | 4.46 | 0.78 | 5.0×10⁷ | 1.3×10⁹ | 1.5×10⁸ |
| | | | | 30 Days | 4.10 | 0.98 | 4.3×10⁷ | 8.1×10⁸ | 7.5×10⁷ |
| 13 | | OLS4803 Strain | | 1 Day | 4.49 | 0.79 | 6.8×10⁷ | 6.3×10⁸ | 5.5×10⁷ |
| | | | | 30 Days | 4.15 | 0.99 | 6.4×10⁷ | 6.7×10⁸ | 8.5×10⁷ |
| 14 | 2038 Strain | OLS4801 Strain | OLS3290 Strain | 1 Day | 4.49 | 0.81 | 1.1×10⁸ | 1.4×10⁹ | 8.0×10⁷ |
| | | | | 30 Days | 4.18 | 0.98 | 4.2×10⁷ | 1.3×10⁹ | 5.5×10⁷ |
| 15 | | OLS4802 Strain | | 1 Day | 4.45 | 0.78 | 1.0×10⁸ | 1.1×10⁹ | 5.0×10⁷ |
| | | | | 30 Days | 4.14 | 0.97 | 4.5×10⁷ | 1.0×10⁹ | 3.0×10⁷ |
| 16 | | OLS4803 Strain | | 1 Day | 4.51 | 0.79 | 1.0×10⁸ | 8.2×10⁸ | 1.0×10⁸ |
| | | | | 30 Days | 4.15 | 0.98 | 4.5×10⁷ | 8.1×10⁸ | 5.0×10⁷ |

### <Comparative Example 1>

First, L. bulgaricus 2038 strain and S. thermophilus 1131 strain not carrying the prtS gene were each activated in the same manner as in Example 1. Next, the milk preparation solution was inoculated with the activated lactic acid bacteria so that the total mass becomes 3 w/w% (L. bulgaricus 2038 strain: 0.3 w/w% (viable cell count: 3.0 × 10⁵ to 9.0 × 10⁷ cfu/g), S. thermophilus 1131 strain: 2.7 w/w% (viable cell count: 2.7 × 10⁶ to 8.1 × 10⁸ cfu/g)); in the same manner as in Example 1, the mixture was fermented by being left to stand at 43°C, and the time (fermentation time) from the inoculation until the pH of the milk preparation solution decreased to reach 4.65 was measured. The fermentation time was 3 hours and 28 minutes. Further, when the pH reached 4.65, the fermentation was terminated to obtain yogurt.

The prepared yogurt was stored in the same manner as in Example 1, the pH (10°C pH) and acidity (lactic acid acidity: %) after 1 day and 30 days from the start of storage were measured, and also the viable cell count (cfu/g) of L. bulgaricus was measured. The results are shown in Table 3 below.

**[Table 3]**

| Comparative Example | L. bulgaricus | prtS+ | prtS- | Storage Period | pH | Acidity (%) | Viable Cell Count for L. bulgaricus (cfu/g) |
|---|---|---|---|---|---|---|---|
| 1 | 2038 Strain | - | 1131 Strain | 1 Day | 4.47 | 0.76 | 1.4×10⁸ |
| | | | | 30 Days | 4.03 | 1.05 | 3.5×10⁶ |

### <Comparative Examples 11 to 12>

Each yogurt was prepared in the same manner as in Comparative Example 1 except that the bacterial strains used in combination as L. bulgaricus and S. thermophilus not carrying the prtS gene (prtS-) were as shown in Table 4 below, and yogurt analysis (measurement of acidity after 1 day and 30 days from the start of storage, and measurement of the viable cell count of L. bulgaricus) was performed. The results are shown in Table 4 below.

**[Table 4]**

| Comparative Example | L. bulgaricus | prtS+ | prtS- | Storage Period | Acidity (%) | Viable Cell Count for L. bulgaricus (cfu/g) |
|---|---|---|---|---|---|---|
| 11 | 1589 Strain | - | OLS3290 Strain | 1 Day | 0.81 | 4.7×10⁷ |
| | | | | 30 Days | 1. 02 | 5.0×10⁶ |
| 12 | 2038 Strain | - | OLS3290 Strain | 1 Day | 0.81 | 8.8×10⁷ |
| | | | | 30 Days | 1.03 | 9.5×10⁶ |

As shown in Tables 3 and 4, in the combination of two lactic acid bacteria (Comparative Examples 1 and 11 to 12), when stored at low temperature, the pH decreased and the acidity significantly increased after 30 days, and the viable cell count of L. bulgaricus also decreased by 10¹ to 10² orders of magnitude. On the other hand, as shown in Tables 1 and 2, according to the combination of three lactic acid bacteria according to the present invention (Examples 1 and 11 to 16), the decrease in pH and the increase in acidity were sufficiently suppressed even after being stored at a low temperature for 30 days, and surprisingly, the viable cell count of L. bulgaricus was also confirmed to be sufficiently maintained at 1 × 10⁷ cfu/g or more. Further, as shown in Tables 2 and 4, this trend was also observed when other bacterial strains were used in combination as L. bulgaricus, S. thermophilus carrying the prtS gene (prtS+), and S. thermophilus not carrying the prtS gene (prtS-).

That is, the combination of three lactic acid bacteria, L. bulgaricus 2038 strain, OLS4802 strain carrying the prtS gene, and 1131 strain not carrying the prtS gene, achieved suppression of decrease in pH and increase in acidity during low-temperature storage, which has been difficult to achieve with the combination of two lactic acid bacteria, 2038 strain and 1131 strain, and the viable cell count of L. bulgaricus (1 × 10⁷ cfu/g or more) . Specifically, in the combination of two lactic acid bacteria, the acidity D₃₀ after 30 days of storage at 10°C was 1.05, and the viable cell count of L. bulgaricus was 3.5 × 10⁶ cfu/g, whereas in the combination of three lactic acid bacteria, D₃₀ was 0.96, and the viable cell count was 2.6 × 10⁷ cfu/g. Further, the fermentation time was further shortened than in the combination of two lactic acid bacteria, and specifically, shortened to 3 hours and 28 minutes (98% or less of the fermentation time for the combination of two lactic acid bacteria) in the case of the combination of two lactic acid bacteria. This shortening can be said to be sufficient for a fermentation time of 3 to 4 hours.

The combination of three lactic acid bacteria, L. bulgaricus 1589 strain, OLS4801 strain carrying the prtS gene, and OLS3290 strain not carrying the prtS gene, achieved suppression of decrease in pH and increase in acidity during low-temperature storage, which has been difficult to achieve with the combination of two lactic acid bacteria, 1589 strain and OLS3290 strain, and the viable cell count of L. bulgaricus (1 × 10⁷ cfu/g or more) . Specifically, in the combination of two lactic acid bacteria, the acidity D₃₀ after 30 days of storage at 10°C was 1.02, and the viable cell count of L. bulgaricus was 5.0 × 10⁶ cfu/g, whereas in the combination of three lactic acid bacteria, D₃₀ was 0.98, and the viable cell count was 3.6 × 10⁷ cfu/g.

The combination of three lactic acid bacteria, L. bulgaricus 1589 strain, OLS4802 strain carrying the prtS gene, and OLS3290 strain not carrying the prtS gene, achieved suppression of decrease in pH and increase in acidity during low-temperature storage, which has been difficult to achieve with the combination of two lactic acid bacteria, 1589 strain and OLS3290 strain, and the viable cell count of L. bulgaricus (1 × 10⁷ cfu/g or more) . Specifically, in the combination of two lactic acid bacteria, the acidity D₃₀ after 30 days of storage at 10°C was 1.02, and the viable cell count of L. bulgaricus was 5.0 × 10⁶ cfu/g, whereas in the combination of three lactic acid bacteria, D₃₀ was 0.98, and the viable cell count was 4.3 × 10⁷ cfu/g.

The combination of three lactic acid bacteria, L. bulgaricus 1589 strain, OLS4803 strain carrying the prtS gene, and OLS3290 strain not carrying the prtS gene, achieved suppression of decrease in pH and increase in acidity during low-temperature storage, which has been difficult to achieve with the combination of two lactic acid bacteria, 1589 strain and OLS3290 strain, and the viable cell count of L. bulgaricus (1 × 10⁷ cfu/g or more). Specifically, in the combination of two lactic acid bacteria, the acidity D₃₀ after 30 days of storage at 10°C was 1.02, and the viable cell count of L. bulgaricus was 5.0 × 10⁶ cfu/g, whereas in the combination of three lactic acid bacteria, D₃₀ was 0.99, and the viable cell count was 6.4 × 10⁷ cfu/g.

The combination of three lactic acid bacteria, L. bulgaricus 2038 strain, OLS4801 strain carrying the prtS gene, and OLS3290 strain not carrying the prtS gene, achieved suppression of decrease in pH and increase in acidity during low-temperature storage, which has been difficult to achieve with the combination of two lactic acid bacteria, 2038 strain and OLS3290 strain, and the viable cell count of L. bulgaricus (1 × 10⁷ cfu/g or more) . Specifically, in the combination of two lactic acid bacteria, the acidity D₃₀ after 30 days of storage at 10°C was 1.03, and the viable cell count of L. bulgaricus was 9.5 × 10⁶ cfu/g, whereas in the combination of three lactic acid bacteria, D₃₀ was 0.98, and the viable cell count was 4.2 × 10⁷ cfu/g.

The combination of three lactic acid bacteria, L. bulgaricus 2038 strain, OLS4802 strain carrying the prtS gene, and OLS3290 strain not carrying the prtS gene, achieved suppression of decrease in pH and increase in acidity during low-temperature storage, which has been difficult to achieve with the combination of two lactic acid bacteria, 2038 strain and OLS3290 strain, and the viable cell count of L. bulgaricus (1 × 10⁷ cfu/g or more) . Specifically, in the combination of two lactic acid bacteria, the acidity D₃₀ after 30 days of storage at 10°C was 1.03, and the viable cell count of L. bulgaricus was 9.5 × 10⁶ cfu/g, whereas in the combination of three lactic acid bacteria, D₃₀ was 0.97, and the viable cell count was 4.5 × 10⁷ cfu/g.

The combination of three lactic acid bacteria, L. bulgaricus 2038 strain, OLS4803 strain carrying the prtS gene, and OLS3290 strain not carrying the prtS gene, achieved suppression of decrease in pH and increase in acidity during low-temperature storage, which has been difficult to achieve with the combination of two lactic acid bacteria, 2038 strain and OLS3290 strain, and the viable cell count of L. bulgaricus (1 × 10⁷ cfu/g or more) . Specifically, in the combination of two lactic acid bacteria, the acidity D₃₀ after 30 days of storage at 10°C was 1.03, and the viable cell count of L. bulgaricus was 9.5 × 10⁶ cfu/g, whereas in the combination of three lactic acid bacteria, D₃₀ was 0.98, and the viable cell count was 4.5 × 10⁷ cfu/g.

### <Example 2>

First, L. bulgaricus OLL205013 strain, S. thermophilus OLS4802 strain carrying the prtS gene, and S. thermophilus OLS3290 strain not carrying the prtS gene were activated in the same manner as in Example 1, respectively. Next, the milk preparation solution was inoculated with the activated lactic acid bacteria so that the total mass becomes 3 w/w% (L. bulgaricus OLL205013 strain: 0.3 w/w% (viable cell count: 3.0 × 10⁵ to 9.0 × 10⁷ cfu/g), S. thermophilus OLS4802 strain: 0.6 w/w% (viable cell count: 6.0 × 10⁵ to 1.8 × 10⁸ cfu/g), S. thermophilus OLS3290 strain: 2.1 w/w% (viable cell count: 2.1 × 10⁶ to 6.3 × 10⁸ cfu/g)); in the same manner as in Example 1, the mixture was fermented by being left to stand at 43°C, and the time from the inoculation (fermentation time) until the pH of the milk preparation solution decreased and reached 4.65 was measured. Further, when the pH reached 4.65, the fermentation was terminated to obtain yogurt. The prepared yogurt was stored in the same manner as in Example 1, and the viable cell count (cfu/g) of L. bulgaricus and the viable cell counts (cfu/g) of each S. thermophilus after 1 day from the start of storage were measured. The results are shown in Table 5 below.

**[Table 5]**

| Example | L. bulgaricus | prtS+ | prtS- | Fermentation Time | Viable Cell Count (cfu/g) | | |
|---|---|---|---|---|---|---|---|
| | | | | | L. bulgaricus | prtS+ | prtS- |
| 2 | OLL205013 Strain | OLS4802 Strain | OLS3290 Strain | 4 h 26 min | 2.0×10⁷ | 6.7×10⁸ | 1.4×10⁸ |

### <Examples 21 to 27>

Fermentation was carried out in the same manner as in Example 2, except that the combinations of bacterial strains shown in Table 6 below were used as L. bulgaricus and S. thermophilus not carrying the prtS gene (prtS-), and the fermentation time was measured. Also, in the same manner as in Example 2, the viable cell count (cfu/g) of L. bulgaricus and the viable cell counts (cfu/g) of each S. thermophilus were measured for each yogurt after 1 day from the start of storage. The results are shown in Table 6 below.

**[Table 6]**

| Example | L. bulgaricus | prtS+ | prtS- | Fermentation Time | Viable Cell Count (cfu/g) | | |
|---|---|---|---|---|---|---|---|
| | | | | | L. bulgaricus | prtS+ | prtS- |
| 21 | OLL1073R-1 Strain | OLS4802 Strain | OLS3290 Strain | 3 h 10 min | 1.4×10⁸ | 1.5×10⁸ | 6.5×10⁷ |
| 22 | OLL1251 Strain | | | 3 h 33 min | 1.1×10⁸ | 4.9×10⁸ | 5.2×10⁸ |
| 23 | OLL1171 Strain | OLS4801 Strain | OLS3615 Strain | 3 h 00 min | 3.0×10⁷ | 7.9×10⁸ | 2.0×10⁷ |
| 24 | | | OLS3078 Strain | 3 h 13 min | 3.0×10⁷ | 8.9×10⁸ | 4.0×10⁷ |
| 25 | OLL1247 Strain | | OLS3290 Strain | 3 h 22 min | 1.0×10⁸ | 1.0×10⁹ | 4.9×10⁷ |
| 26 | | | OLS3078 Strain | 3 h 14 min | 1.1×10⁸ | 9.1×10⁸ | 6.1×10⁷ |
| 27 | OLL1251 Strain | | OLS3078 Strain | 3 h 29 min | 4.9×10⁷ | 8.6×10⁸ | 3.4×10⁷ |

### <Comparative Example 2>

First, L. bulgaricus OLL205013 strain and S. thermophilus OLS3290 strain not carrying the prtS gene were activated in the same manner as in Example 1, respectively. Next, the milk preparation solution was inoculated with the activated lactic acid bacteria so that the total mass becomes 3 w/w% (L. bulgaricus OLL205013 strain: 0.3 w/w% (viable cell count: 3.0 × 10⁵ to 9.0 × 10⁷ cfu/g), S. thermophilus OLS3290 strain: 2.7 w/w% (viable cell count: 2.7 × 10⁶ to 8.1 × 10⁸ cfu/g)); in the same manner as in Example 1, the mixture was fermented by being left to stand at 43°C, and the time from the inoculation (fermentation time) until the pH of the milk preparation solution decreased and reached 4.65 was measured. Further, when the pH reached 4.65, the fermentation was terminated to obtain yogurt. The prepared yogurt was stored in the same manner as in Example 1, and the viable cell count of L. bulgaricus (cfu/g) at 1 day from the start of storage was measured. The results are shown in Table 7 below.

**[Table 7]**

| Comparative Example | L. bulgaricus | prtS+ | prtS- | Fermentation Time | Viable Cell Count for L. bulgaricus (cfu/g) |
|---|---|---|---|---|---|
| 2 | OLL205013 Strain | - | OLS3290 Strain | 7 h 16 min | 2.3×10⁷ |

### <Comparative Examples 21 to 23>

Fermentation was performed in the same manner as in Comparative Example 2 except that the bacterial strains shown in Table 8 below were used in combination as L. bulgaricus and S. thermophilus not carrying the prtS gene (prtS-), and the fermentation time was measured. Further, in the same manner as in Comparative Example 2, for each yogurt, the viable cell count of L. bulgaricus (cfu/g) and the viable cell count of each S. thermophilus (cfu/g) at 1 day from the start of storage were measured. The results are shown in Table 8 below.

**[Table 8]**

| Comparative Example | L. bulgaricus | prtS+ | prtS- | Fermentation Time | Viable Cell Count for L. bulgaricus (cfu/g) |
|---|---|---|---|---|---|
| 21 | OLL1073R-1 Strain | - | OLS3290 Strain | 4 h 03 min | 2.1×10⁸ |
| 22 | OLL1251 Strain | | | 4 h 02 min | 1.3×10⁸ |
| 23 | OLL1247 Strain | | | 4 h 08 min | 1.9×10⁸ |

As shown in Tables 5 and 7, the fermentation time was greatly reduced to about 0.6 times, about 4 hours, by using the combination of three lactic acid bacteria according to the present invention (Example 2), as compared to the combination of two lactic acid bacteria having a long fermentation time of 7 hours or more (Comparative Example 2), thus achieving the shortening of the fermentation time that was difficult to achieve with the combination of two lactic acid bacteria. Furthermore, in the combination of the three lactic acid bacteria according to the present invention, despite the shortening of the fermentation time, the viable cell count of L. bulgaricus after fermentation was able to reach the same level as that of the combination of two lactic acid bacteria having a long fermentation time. Further, as shown in Tables 6 and 8, even when various combinations were used as L. bulgaricus, S. thermophilus carrying the prtS gene, and S. thermophilus not carrying the prtS gene, the fermentation time was shortened compared to the combination of two lactic acid bacteria, and the viable cell count of L. bulgaricus after fermentation was also sufficiently maintained.

### <Example 3>

First, using L. bulgaricus 2038 strain, S. thermophilus OLS4802 strain carrying the prtS gene, and S. thermophilus 1131 strain not carrying the prtS gene, yogurt was prepared in the same manner as in Example 1. The prepared yogurt was immediately cooled with stirring and the temperature was lowered to 10°C; then, by adding lactic acid (food additive) and mixing with stirring, the pH was artificially adjusted to 4.5. The yogurt at pH 4.5 was sealed so as not to allow air to enter, stored by being left to stand at 10°C, and the viable cell count (cfu/g) of L. bulgaricus at each of pH 4.5, pH 4.3, pH 4.1, and pH 4.0 was measured by the method described in Example 1. Table 9 below shows the relative viable cell count of L. bulgaricus (cfu/g) at each pH, with the viable cell count of L. bulgaricus (cfu/g) at pH 4.5 being taken as 1.0.

**[Table 9]**

| pH | Viable Cell Count for L. bulgaricus vs. pH4.5 (cfu/g) |
|---|---|
| 4.5 | 1.00 |
| 4.3 | 0.76 |
| 4.1 | 0.10 |
| 4.0 | 0.02 |

### <Comparative Example 3>

Using L. bulgaricus 2038 strain and S. thermophilus 1131 strain not carrying the prtS gene, yogurt was prepared in the same manner as in Comparative Example 1. The prepared yogurt was immediately cooled with stirring and the temperature was lowered to 10°C; then, by adding lactic acid (food additive) and mixing with stirring, the pH was artificially adjusted to 4.5. The yogurt at pH 4.5 was sealed so as not to allow air to enter, stored by leaving to stand at 10°C, and the viable cell count (cfu/g) of L. bulgaricus at each time point of pH 4.5, pH 4.3, pH 4.1, and pH 4.0 was measured by the method described in Example 1. Table 10 shows the relative viable cell count of L. bulgaricus (cfu/g) at each pH, with the viable cell count (cfu/g) of L. bulgaricus at the time of pH 4.5 being 1.0.

**[Table 10]**

| pH | Viable Cell Count for L. bulgaricus vs. pH4.5 (cfu/g) |
|---|---|
| 4.5 | 1.00 |
| 4.3 | 0.66 |
| 4.1 | 0.08 |
| 4.0 | < 0.01 |

As shown in Tables 9 and 10, according to the combination of three lactic acid bacteria of the present invention (Example 3), even when the pH was artificially decreased to 4.5 and stored at a low temperature, and the pH was further decreased to 4.0, the viable cell count of L. bulgaricus was maintained at a higher level than in the combination of two lactic acid bacteria at the same pH (Comparative Example 3).

### <Example 4>

### (4-1 to 4-3)

First, L. bulgaricus 2038 strain, S. thermophilus OLS4802 strain carrying the prtS gene, and S. thermophilus 1131 strain not carrying the prtS gene were activated in the same manner as in Example 1. Next, the milk preparation solution was inoculated with the activated lactic acid bacteria so that the total mass becomes 3 w/w% (L. bulgaricus 2038 strain: 0.3 w/w% (viable cell count: 3.0 × 10⁵ to 9.0 × 10⁷ cfu/g), S. thermophilus OLS4802 strain: 1.35 w/w% (viable cell count: 1.4 × 10⁶ to 4.2 × 10⁸ cfu/g), S. thermophilus 1131 strain: 1.35 w/w% (viable cell count: 1.4 × 10⁶ to 4.2 × 10⁸ cfu/g)), and fermented by leaving to stand at 43°C in the same manner as in Example 1. When the pH of the milk preparation solution decreased and reached 4.65, the fermentation was terminated to obtain Yogurt 4-1.

Yogurt 4-2 was prepared in the same manner as in Yogurt 4-1 except that L. bulgaricus 2038 strain, S. thermophilus OLS4802 strain, and S. thermophilus OLS3290 strain not carrying the prtS gene, which was determined to be a high polysaccharide-producing lactic acid bacterium in this example, were used. Further, Yogurt 4-3 was prepared in the same manner as Yogurt 4-1, except that L. bulgaricus OLL1251 strain, which was determined to be a high polysaccharide-producing lactic acid bacterium in this example, S. thermophilus OLS4802 strain, and S. thermophilus OLS3290 strain were used. Table 11 below shows the lactic acid bacteria combinations in Yogurts 4-1 to 4-3.

**[Table 11]**

| Yogurt | L. bulgaricus | prtS+ | prtS- |
|---|---|---|---|
| 4-1 | 2038 Strain | OLS4802 Strain | 1131 Strain |
| 4-2 | 2038 Strain | OLS4802 Strain | OLS3290 Strain |
| 4-3 | OLL1251 Strain | OLS4802 Strain | OLS3290 Strain |

The prepared Yogurts 4-1 to 4-3 were subjected to sensory evaluation of viscosity by a total of 10 panelists. All the panelists were experts (sensory evaluation experts) who had undergone sensory evaluation training at Meiji Co., Ltd., which manufactures and sells fermented milk, and have more than 10 years of experience in conducting sensory evaluation tests in their daily work. As a result of evaluating Yogurt 4-1 and Yogurt 4-2 by the two-point test method according to JIS Z 9080, 10 out of 10 panelists evaluated that the viscosity of Yogurt 4-2 was higher, and it was determined that the panelists could significantly discriminate between them at a significance level of 5%. Furthermore, as a result of evaluating Yogurt 4-2 and Yogurt 4-3 by the two-point test method, 10 out of 10 panelists evaluated that Yogurt 4-3 was higher in viscosity, and it was determined that the panelists could significantly discriminate them at a significance level of 5%. From this, it was confirmed that in the combination of three lactic acid bacteria according to the present invention, the viscosity of yogurt can be easily adjusted by using a high polysaccharide-producing lactic acid bacterium that produces more polysaccharides as L. bulgaricus and/or S. thermophilus not carrying the prtS gene.

### (4-4 to 4-6)

Each yogurt was prepared in the same manner as Yogurts 4-1 to 4-3, respectively, except that the bacterial strains shown in Table 12 below were used in combination as L. bulgaricus, S. thermophilus carrying the prtS gene (prtS+), and S. thermophilus not carrying the prtS gene (prtS-).

**[Table 12]**

| Yogurt | L. bulgaricus | prtS+ | prtS- |
|---|---|---|---|
| 4-4 | 1589 Strain | OLS4802 Strain | OLS3078 Strain |
| 4-5 | 1589 Strain | OLS4802 Strain | OLS3290 Strain |
| 4-6 | OLL1073R-1 Strain | OLS4802 Strain | OLS3290 Strain |

The prepared Yogurts 4-4 to 4-6 were subjected to sensory evaluation of viscosity by a total of 5 panelists. All the panelists were experts (sensory evaluation experts) who had undergone sensory evaluation training at Meiji Co., Ltd., which manufactures and sells fermented milk, and have more than 10 years of experience in conducting sensory evaluation tests in their daily work. As a result of evaluating Yogurt 4-4 and Yogurt 4-5 by the two-point test method according to JIS Z 9080, 5 out of 5 panelists evaluated that Yogurt 4-5 was higher in viscosity, and it was determined that the panelists could significantly discriminate between them at a significance level of 5%. Furthermore, as a result of evaluating Yogurt 4-5 and Yogurt 4-6 by the two-point test method, 5 out of 5 panelists evaluated that Yogurt 4-6 was higher in viscosity, and it was determined that the panelists could significantly discriminate them at a significance level of 5%. From this, it was confirmed that even when other bacterial strains were used, in the combination of three lactic acid bacteria according to the present invention, the viscosity of yogurt can be easily adjusted by using a high polysaccharide-producing lactic acid bacterium that produces more polysaccharides as L. bulgaricus and/or S. thermophilus not carrying the prtS gene.

### <Example 5>

First, L. bulgaricus 2038 strain, S. thermophilus OLS4802 strain carrying the prtS gene, and S. thermophilus OLS3290 strain not carrying the prtS gene were activated in the same manner as in Example 1. Next, the milk preparation solution was inoculated with the activated lactic acid bacteria so that the total mass becomes 3 w/w% (L. bulgaricus 2038 strain: 0.3 w/w% (viable cell count: 3.0 × 10⁵ to 9.0 × 10⁷ cfu/g), S. thermophilus OLS4802 strain: 1.35 w/w% (viable cell count: 1.4 × 10⁶ to 4.2 × 10⁸ cfu/g), S. thermophilus OLS3290 strain: 1.35 w/w% (viable cell count: 1.4 × 10⁶ to 4.2 × 10⁸ cfu/g)), and fermented by leaving to stand at 43°C in the same manner as in Example 1. When the pH of the milk preparation solution decreased and reached 4.65, the fermentation was terminated to obtain Yogurt 5-1.

Yogurt 5-2 was prepared in the same manner as Yogurt 5-1 except that L. bulgaricus 2038 strain, S. thermophilus OLS4802 strain carrying the prtS gene, and S. thermophilus 1131 strain not carrying the prtS gene were used. Further, Yogurt 5-3 was prepared in the same manner as Yogurt 5-1 except that L. bulgaricus 2038 strain, S. thermophilus OLS4802 strain carrying the prtS gene, and S. thermophilus OLS3078 strain not carrying the prtS gene were used.

The prepared yogurt was stored in the same manner as in Example 1. 40 g each of yogurt after 5 days and 20 days from the start of storage was weighed and centrifuged at 1,610 × g and 25°C for 10 minutes, and the weight of the precipitate after removing the supernatant was measured; then, the syneresis rate (%) was calculated by the following formula: weight of precipitate (g)/40 g × 100. Table 13 below shows the combinations of lactic acid bacteria and the syneresis rate values in Yogurts 5-1 to 5-3.

### <Comparative Example 5>

First, L. bulgaricus 2038 strain and S. thermophilus OLS4802 strain carrying the prtS gene were activated in the same manner as in Example 1. Next, the milk preparation solution was inoculated with the activated lactic acid bacteria so that the total mass becomes 3 w/w% (L. bulgaricus 2038 strain: 0.3 w/w% (viable cell count: 3.0 × 10⁵ to 9.0 × 10⁷ cfu/g), S. thermophilus OLS4802 strain: 2.7 w/w% (viable cell count: 2.7 × 10⁶ to 8.1 × 10⁸ cfu/g)), and fermented by leaving to stand at 43°C in the same manner as in Example 1. When the pH of the milk preparation solution decreased and reached 4.65, fermentation was stopped to obtain yogurt. For the prepared yogurt, the syneresis rate (%) at 5 days and 20 days from the start of storage was calculated in the same manner as in Example 5. Table 13 below also shows the combinations of lactic acid bacteria and syneresis rate values in the prepared yogurt.

**[Table 13]**

| Yogurt | | L. bulgaricus | prtS+ | prtS- | Syneresis Rate (%) at Day 5 of Storage | Syneresis Rate (%) at Day 20 of Storage |
|---|---|---|---|---|---|---|
| Example | 5-1 | 2038 Strain | OLS4802 Strain | OLS3290 Strain | 22.4 | 26.9 |
| | 5-2 | | | 1131 Strain | 23.2 | 29.0 |
| | 5-3 | | | OLS3078 Strain | 22.7 | 27.4 |
| Comparative Example | 5 | | | - | 23.7 | 31.2 |

As shown in Table 13, it was confirmed that the combination of three lactic acid bacteria according to the present invention (Example 5, Yogurts 5-1 to 5-3) had lower syneresis rate even after 20 days of storage at low temperature, compared to the combination of two lactic acid bacteria (Comparative Example 5). In order to confirm this also by sensory evaluation, the prepared Yogurts 5-1 to 5-3 and the yogurt of Comparative Example 5 were evaluated for smoothness by a total of 10 or 8 panelists. All of the panelists were experts (sensory evaluation experts) who had undergone sensory evaluation training at Meiji Co., Ltd., which manufactures and sells fermented milk, and had more than 10 years of experience in conducting sensory evaluation tests in their daily work. As a result of evaluating the yogurt of Comparative Example 5 and Yogurt 5-1 by the two-point discrimination method according to JIS Z 9080, 10 out of 10 panelists evaluated that Yogurt 5-1 was smoother, and it was determined that the panelists could discriminate significantly at a 5% significance level. Also, as a result of evaluating the yogurt of Comparative Example 5 and Yogurt 5-2 by the two-point discrimination method, 8 out of 8 panelists evaluated that Yogurt 5-2 was smoother, and it was determined that the panelists could significantly discriminate them at a significance level of 5%. Further, as a result of evaluating the yogurt of Comparative Example 5 and Yogurt 5-3 by the two-point discrimination method, 10 out of 10 panelists evaluated that Yogurt 5-3 was smoother, and it was determined that the panelists could significantly discriminate them at a significance level of 5%. From the above, it was confirmed that the combination of the three lactic acid bacteria according to the present invention can provide yogurt (fermented milk) having smooth physical properties.

### <Examples 51 to 57, Comparative Examples 51 to 57>

Each yogurt of Examples 51 to 57 was prepared in the same manner as Yogurt 5-1 of Example 5 except using the bacterial strains shown in Table 14 below as respective combinations of L. bulgaricus, S. thermophilus carrying the prtS gene (prtS+), and S. thermophilus not carrying the prtS gene (prtS-). Each yogurt of Comparative Examples 51 to 57 was prepared in the same manner as the yogurt of Comparative Example 5 except using the bacterial strains shown in Table 14 below as respective combinations of L. bulgaricus and S. thermophilus carrying the prtS gene (prtS+). For each of the prepared yogurts, the syneresis rate (%) at 5 days and 20 days from the start of storage was calculated in the same manner as in Example 5. Table 14 below also shows the combinations of lactic acid bacteria and the syneresis rate values of each of the prepared yogurts.

As shown in Table 14, similar to Example 5, even when other bacterial strains were used, it was confirmed that the combination of three lactic acid bacteria according to the present invention (Examples 51 to 57) had lower syneresis rate even after 20 days of storage at low temperature compared to the combination of two lactic acid bacteria (Comparative Examples 51 to 57). Further, as a result of evaluating the smoothness by a total of 5 panelists in the same manner as described above, 5 out of 5 panelists evaluated that the yogurts of Examples 51 to 57 were smoother than the yogurts of Comparative Examples 51 to 57, respectively, and it was determined that the panelists were able to discriminate significantly at a significance level of 5%. All of the panelists were experts (sensory evaluation experts) who had undergone sensory evaluation training at Meiji Co., Ltd., which manufactures and sells fermented milk, and had more than 10 years of experience in conducting sensory evaluation tests in their daily work. From this, it was confirmed that even when other bacterial strains are used, the combination of three lactic acid bacteria according to the present invention can provide yogurt (fermented milk) having smooth physical properties.

### [Industrial Applicability]

As described above, the present invention makes it possible to provide fermented milk in which the viable

**[Table 14]**

| Yogurt | | L. bulgaricus | prtS+ | prtS- | Syneresis Rate (%) at Day 5 of Storage | Syneresis Rate (%) at Day 20 of Storage |
|---|---|---|---|---|---|---|
| Example | 51 | 1589 Strain | OLS4801 Strain | OLS3290 Strain | 23.0 | 23.5 |
| Comparative Example | 51 | | | - | 26.0 | 35.3 |
| Example | 52 | | OLS4802 Strain | OLS3290 Strain | 23.3 | 24.8 |
| Comparative Example | 52 | | | - | 23.0 | 35.0 |
| Example | 53 | | OLS4803 Strain | OLS3290 Strain | 24.7 | 26.1 |
| Comparative Example | 53 | | | - | 23.9 | 33.8 |
| Example | 54 | 2038 Strain | OLS4801 Strain | OLS3290 Strain | 22.7 | 24.3 |
| Comparative Example | 54 | | | - | 24.8 | 34.0 |
| Example | 55 | | OLS4803 Strain | OLS3290 Strain | 21.1 | 23.5 |
| Comparative Example | 55 | | | - | 25.8 | 35.9 |
| Example | 56 | OLL205013 Strain | OLS4801 Strain | OLS3078 Strain | 23.3 | 28.6 |
| Comparative Example | 56 | | | - | 25.5 | 36.0 |
| Example | 57 | | OLS4803 Strain | OLS3078 Strain | 22.9 | 28.9 |
| Comparative Example | 57 | | | - | 24.7 | 36.2 |

cell count of Lactobacillus delbrueckii subsp. bulgaricus is sufficiently maintained even after low-temperature storage, the decrease in pH and the increase in acidity during low-temperature storage are also suppressed, and which has smooth physical properties, and a lactic acid bacteria composition and a method for producing fermented milk that can obtain the fermented milk in a sufficiently short fermentation time.

### [Accession Number]

1.
   (1) Identification label: Lactobacillus delbrueckii subsp. bulgaricus OLL1171
   (2) Accession number: NITE BP-01569
   (3) Date of accession: March 13, 2013
   (4) Depositary institution: NITE Patent Microorganisms Depositary, National Institute of Technology and Evaluation
2.
   (1) Identification label: Lactobacillus delbrueckii subsp. bulgaricus OLL1073R-1
   (2) Accession number: FERM BP-10741
   (3) Date of accession: February 22, 1999
   (4) Depositary institution: International Patent Organism Depositary, National Institute of Advanced Industrial Science and Technology (currently: International Patent Organism Depositary, National Institute of Technology and Evaluation)
3.
   (1) Identification label: Lactobacillus delbrueckii subsp. bulgaricus OLL205013
   (2) Accession number: NITE BP-02411
   (3) Date of accession: February 3, 2017
   (4) Depositary institution: NITE Patent Microorganisms Depositary, National Institute of Technology and Evaluation
4.
   (1) Identification label: Lactobacillus delbrueckii subsp. bulgaricus OLL1247
   (2) Accession number: NITE BP-01814
   (3) Date of accession: March 6, 2014
   (4) Depositary institution: NITE Patent Microorganisms Depositary, National Institute of Technology and Evaluation
5.
   (1) Identification label: Lactobacillus delbrueckii subsp. bulgaricus OLL1251
   (2) Accession number: NITE BP-02703
   (3) Date of accession: April 25, 2018
   (4) Depositary institution: NITE Patent Microorganisms Depositary, National Institute of Technology and Evaluation
6.
   (1) Identification label: Streptococcus thermophilus OLS4801
   (2) Accession number: NITE BP-03504
   (3) Date of accession: August 4, 2021
   (4) Depositary institution: NITE Patent Microorganisms Depositary, National Institute of Technology and Evaluation
7.
   (1) Identification label: Streptococcus thermophilus OLS4802
   (2) Accession number: NITE BP-03505
   (3) Date of accession: August 4, 2021
   (4) Depositary institution: NITE Patent Microorganisms Depositary, National Institute of Technology and Evaluation
8.
   (1) Identification label: Streptococcus thermophilus OLS4803
   (2) Accession number: NITE BP-03506
   (3) Date of accession: August 4, 2021
   (4) Depositary institution: NITE Patent Microorganisms Depositary, National Institute of Technology and Evaluation
9.
   (1) Identification label: Streptococcus thermophilus OLS4823
   (2) Accession number: NITE BP-03507
   (3) Date of accession: August 4, 2021
   (4) Depositary institution: NITE Patent Microorganisms Depositary, National Institute of Technology and Evaluation
10.
   (1) Identification label: Streptococcus thermophilus OLS4824
   (2) Accession number: NITE BP-03508
   (3) Date of accession: August 4, 2021
   (4) Depositary institution: NITE Patent Microorganisms Depositary, National Institute of Technology and Evaluation
11.
   (1) Identification label: Streptococcus thermophilus OLS3078
   (2) Accession number: NITE BP-01697
   (3) Date of accession: August 23, 2013
   (4) Depositary institution: NITE Patent Microorganisms Depositary, National Institute of Technology and Evaluation
12.
   (1) Identification label: Streptococcus thermophilus OLS3615
   (2) Accession number: NITE BP-01696
   (3) Date of accession: August 23, 2013
   (4) Depositary institution: NITE Patent Microorganisms Depositary, National Institute of Technology and Evaluation
13.
   (1) Identification label: Streptococcus thermophilus OLS3290
   (2) Accession number: FERM BP-19638
   (3) Date of accession: January 19, 2004
   (4) Depositary institution: International Patent Organism Depositary, National Institute of Technology and Evaluation
14.
   (1) Identification label: Lactobacillus delbrueckii subsp. bulgaricus 1589
   (2) Accession number: NITE BP-03716
   (3) Date of accession: August 9, 2022
   (4) Depositary institution: NITE Patent Microorganisms Depositary, National Institute of Technology and Evaluation

## Claims

1. A lactic acid bacteria composition comprising: Lactobacillus delbrueckii subsp. bulgaricus; Streptococcus thermophilus carrying a prtS gene; and Streptococcus thermophilus not carrying a prtS gene.

2. The lactic acid bacteria composition according to claim 1, wherein a content of the Lactobacillus delbrueckii subsp. bulgaricus and a total content of the Streptococcus thermophilus carrying the prtS gene and the Streptococcus thermophilus not carrying the prtS gene are in a mass ratio of 1:1 to 1:150.

3. The lactic acid bacteria composition according to claim 1, wherein a content of the Streptococcus thermophilus carrying the prtS gene and a content of the Streptococcus thermophilus not carrying the prtS gene are in a mass ratio of 1:0.05 to 1:10.

4. The lactic acid bacteria composition according to claim 1, which is a composition in which, when the composition is added to a milk preparation solution comprising raw material milk and fermented at 43°C until a pH of the milk preparation solution reaches 4.65 and the post-fermentation composition is stored at 10°C for 30 days, a viable cell count of Lactobacillus delbrueckii subsp. bulgaricus in the post-fermentation composition at 30 days of storage becomes 1 × 10⁷ cfu/g or more.

5. The lactic acid bacteria composition according to claim 1, which is a composition in which, when the composition is added to a milk preparation solution comprising raw material milk and fermented at 43°C until a pH of the milk preparation solution reaches 4.65 and the post-fermentation composition is stored at 10°C for 30 days, an acidity of the post-fermentation composition at 30 days of storage is 1.0% or less.

6. The lactic acid bacteria composition according to claim 1, which is a composition in which, when the composition is added to a milk preparation solution comprising raw material milk and fermented at 43°C until a pH of the milk preparation solution reaches 4.65 and the post-fermentation composition is stored at 10°C for 30 days, an acidity (D₁%) of the post-fermentation composition at 1 day of storage and an acidity (D₃₀%) of the post-fermentation composition at 30 days of storage satisfy a condition expressed by the following formula: D₃₀ - D₁ ≤ 0.25.

7. The lactic acid bacteria composition according to claim 1, wherein the Lactobacillus delbrueckii subsp. bulgaricus and/or the Streptococcus thermophilus not carrying the prtS gene are/is a high polysaccharide-producing lactic acid bacteria/bacterium.

8. The lactic acid bacteria composition according to claim 1, which is a lactic acid bacteria starter.

9. The lactic acid bacteria composition according to claim 1, which is a fermented milk.

10. A lactic acid bacteria combination comprising: Lactobacillus delbrueckii subsp. bulgaricus; Streptococcus thermophilus carrying a prtS gene; and Streptococcus thermophilus not carrying a prtS gene.

11. The lactic acid bacteria combination according to claim 10, which is a lactic acid bacteria starter.

12. A method for producing fermented milk, comprising a fermentation step of adding the lactic acid bacteria composition according to any one of claims 1 to 9 or the lactic acid bacteria combination according to claim 10 or 11 to a milk preparation solution comprising raw material milk and fermenting the mixture to obtain a fermented milk.
